# EUROPEAN PATENT APPLICATION

(11) **EP 4 613 863 A1**
(43) Date of publication of application: **10.09.2025**
(21) Application number: 23884972.3
(22) Date of filing: 01.11.2023
(51) Int. Cl.: C12N 15/53, C12N 9/02, C12N 15/864, C12N 7/01, A61K 48/00, A61P 3/00

(54) **OPTIMIZED PAH GENE AND EXPRESSION CASSETTE AND USE THEREOF**

(30) Priority: 02.11.2022 CN 202211364551
(71) Applicant: Suzhou NGGT Biotechnology Co.,Ltd., Suzhou, Jiangsu 215000 (CN)
(72) Inventor: WEN, Shengmei, Suzhou, Jiangsu 215000 (CN); LIU, Yiting, Suzhou, Jiangsu 215000 (CN); GUO, Yufeng, Suzhou, Jiangsu 215000 (CN); JIANG, Lixin, Suzhou, Jiangsu 215000 (CN)
(74) Representative: Hoffmann Eitle
(86) International application number: PCT/CN2023/128981
(87) International publication number: WO 2024/094044

(57) **Abstract**

The present disclosure relates to an optimized PAH gene and an expression cassette and use thereof. Specifically, a polynucleotide molecule encoding PAH protein is disclosed. By means of using the polynucleotide molecule, expression cassette, expression vector, virion, and/or pharmaceutical composition provided herein, human PAH can be effectively, persistently, and stably expressed in the liver at a relatively low dose, thus steadily keeping the blood phenylalanine concentration in a subject at a relatively low level for a long period. Therefore, the present disclosure can be used for the treatment of phenylketonuria.

## Description

### Technical Field

The present disclosure belongs to the technical field of gene therapy, and particularly relates to an optimized PAH gene and expression cassette and uses thereof.

### Background Art

Phenylketonuria (PKU, OMIM 261600) is an autosomal recessive genetic disease caused by mutations in the phenylalanine hydroxylase (PAH) gene. The incidence rate in the United States is 1/13,500, and the incidence rate in China is approximately 1/15,924. In neonates and children, the concentration of peripheral blood phenylalanine can be well managed through low-phenylalanine infant formula and diet, thereby ensuring the development of the nervous system. Currently, oral tetrahydrobiopterin, also known as sapropterin (brand name as Kuvan), and subcutaneous long-acting phenylalanine ammonia lyase (pegvaliase-pqpz, brand name as Palynziq, a PEGylated phenylalanine ammonia lyase) are available for use in adults. However, there are disadvantages and limitations in both drugs, mainly manifested in that they are suitable only for a subset of patients, cause immune-related side effects, and have a slow onset of action, etc.

According to the current genetic variation classification standards and treatment guidelines in United States, it is recommended to maintain the peripheral blood phenylalanine concentration between 120 µmol/L and 360 µmol/L via dietary management and medication throughout life (Vockley, J., et al., Phenylalanine hydroxylase deficiency: diagnosis and management guideline. Genet Med, 2014. 16 (2): p. 188-200.). The control standard of the EU treatment guidelines is less than 360 µmol/L for women under 12 years old, preparing for pregnancy or pregnant, and less than 600 µmol/L for other population (van Wegberg, AMJ, et al., The complete European guidelines on phenylketonuria: diagnosis and treatment. Orphanet J Rare Dis, 2017. 12 (1): p. 162.). However, since it is difficult for adult patients to adhere to dietary control, only about 19% of patients do not adhere to it for more than 9 months. Therefore, most adult patients lose follow-up and have hyperphenylalaninemia. A survey showed that among adult PKU patients, 67% exhibited phenylalanine concentrations (Phe)>360 µmol/L, 45% exceeded 600 µmol/L, and 18% exceeded 1200 µmol/L. Only about 24% of adult patients maintained concentrations at ≤360 µmol/L (Brown, CS and U. Lichter-Konecki, Phenylketonuria (PKU): A problem solved? Mol Genet Metab Rep, 2016. 6: p. 8-12.).

Even with good early treatment, adult patients are likely to develop neurological symptoms such as tremor, active deep tendon reflexes, poor motor coordination, and white matter abnormalities, if peripheral blood phenylalanine was not managed. In addition, adult PKU patients also face many quality of life issues, such as low work ability, lack of autonomy, hopelessness, low motivation, depression and anxiety, difficulty in maintaining long-term relationships with friends, and prone to running away from home in old age (Murphy, GH, et al., Adults with untreated phenylketonuria: out of sight, out of mind. Br J Psychiatry, 2008. 193(6): p. 501-2.; Hoeks, MP, M. den Heijer, and MC Janssen, Adult issues in phenylketonuria. Neth J Med, 2009. 67(1): p. 2-7.). For adolescents and adults with late-diagnosed late or inadequate early treatment, high peripheral phenylalanine levels can lead to seizures, spasms, severe behavioral issues such as aggressive behaviors, self-injury, hyperactivity, restlessness, irritability, sleep disturbances, anxiety, stereotypic behaviors, and neurological and cognitive problems (van Vliet, D., et al., Can untreated PKU patients escape from intellectual disability? A systematic review. Orphanet J Rare Dis, 2018. 13 (1): p. 149.; Ashe, K., et al., Psychiatric and Cognitive Aspects of Phenylketonuria: The Limitations of Diet and Promise of New Treatments. Front Psychiatry, 2019. 10: p. 561.; Romani, C., et al., Adult cognitive outcomes in phenylketonuria: explaining causes of variability beyond average Phe levels. Orphanet J Rare Dis, 2019. 14 (1): p. 561. (1): p. 273.; Trepp, R., et al., Impact of phenylalanine on cognitive, cerebral, and neurometabolic parameters in adult patients with phenylketonuria (the PICO study): a randomized, placebo-controlled, crossover, noninferiority trial. Trials, 2020. 21 (1): p. 178.; Altman, G., et al., Mental health diagnoses in adults with phenylketonuria: a retrospective systematic audit in a large UK single centre. Orphanet J Rare Dis, 2021. 16 (1): p. 520.; Trefz, F., et al., Health economic burden of patients with phenylketonuria (PKU) - A retrospective study of German health insurance claims data. Mol Genet Metab Rep, 2021. 27: p. 100764.; Yamada, K., et. al., Long-Term Neurological Outcomes of Adult Patients with Phenylketonuria before and after Newborn Screening in Japan. Int J Neonatal Screen, 2021. 7 (2).).

Therefore, there is still a great clinical need for the treatment of phenylketonuria in adults. Studies have shown that the treatment for adult patients by controlling peripheral blood phenylalanine concentrations can reduce or stop epileptic seizures and improve behavioral and neurocognitive issues (van Spronsen, FJ, et al., Phenylketonuria. Nat Rev Dis Primers, 2021. 7 (1): p. 36.).

After nearly 60 years of development, gene therapy has achieved remarkable results. A single treatment with Zolgensma can allow children with spinal muscular atrophy (SMA) to grow and develop essentially like normal children. A single treatment with Luxturna can restore the vision of patients with Leber congenital amaurosis (LCA) to a state where they can basically live, study and work normally. Globally, in the present, two gene therapy projects for PKU are in Phase I clinical trials. High doses are used in both trials. However, neither has reported effective therapeutic effects yet, but there is a risk of potential liver toxicity associated with high doses.

### Summary of the Invention

The purpose of the present disclosure is providing an optimized PAH gene, expression cassette and viral vector, to achieve effective, durable and stable expression of human PAH in the liver at a relatively low dose for the treatment of phenylketonuria.

To solve the above technical problems, the following technical solutions are provided in the present disclosure:
In a first aspect, the present disclosure provides a polynucleotide molecule encoding a PAH protein, comprising a nucleotide sequence having 90% or more identity with the nucleotide sequence set forth in SEQ ID NO. 10, SEQ ID NO. 11, SEQ ID NO. 12, SEQ ID NO. 13, SEQ ID NO. 14, SEQ ID NO. 15, SEQ ID NO. 16, SEQ ID NO. 17, SEQ ID NO. 19, SEQ ID NO. 20, SEQ ID NO. 21, SEQ ID NO. 22 or SEQ ID NO. 23; preferably a nucleotide sequence having 91%, 92%, 93%, 94%, 95%, 96%, 97%, 98%, 99% or more identity; more preferably a nucleotide sequence having 98% or more identity.

The second aspect of the present disclosure provides an expression cassette, which comprises the polynucleotide molecule provided in the first aspect of the present disclosure, and a promoter operably linked to the polynucleotide molecule.

In some embodiments, the expression cassette further comprises an expression control element, which is operably linked to the polynucleotide molecule.

In some embodiments, the expression control element is selected from at least one of a transcription/translation control signal, an enhancer, an intron, a polyA signal, an ITR, an insulator, an RNA processing signal, and an element that enhances the stability of mRNA and protein.

The third aspect of the present disclosure provides an expression vector, which comprises the polynucleotide molecule provided by the first aspect of the present disclosure or the expression cassette provided by the second aspect of the present disclosure.

In some embodiments, the expression vector is selected from a plasmid, a cosmid, a viral vector, an RNA vector, or a linear or circular DNA or RNA molecule.

In some embodiments, the expression vector is an adeno-associated virus vector.

The fourth aspect of the present disclosure provides a virus particle, which comprises at least one of the polynucleotide molecule provided by the first aspect of the present disclosure, the expression cassette provided by the second aspect of the present disclosure, and the expression vector provided by the third aspect of the present disclosure.

The fifth aspect of the present disclosure provides a pharmaceutical composition for treating phenylketonuria, which comprises at least one of the polynucleotide molecule provided in the first aspect of the present disclosure, the expression cassette provided in the second aspect of the present disclosure, the expression vector provided in the third aspect of the present disclosure, and the virus particle provided in the fourth aspect of the present disclosure.

The sixth aspect of the present disclosure provides the use of at least one of the polynucleotide molecule provided in the first aspect of the present disclosure, the expression cassette provided in the second aspect of the present disclosure, the expression vector provided in the third aspect of the present disclosure, the virus particle provided in the fourth aspect of the present disclosure, and the pharmaceutical composition of the fifth aspect of the present disclosure, in the preparation of a medicament for treating phenylketonuria.

The seventh aspect of the present disclosure provides the use of at least one of the polynucleotide molecule provided in the first aspect of the present disclosure, the expression cassette provided in the second aspect of the present disclosure, the expression vector provided in the third aspect of the present disclosure, the viral particle mentioned in the fourth aspect of the present disclosure, and the pharmaceutical composition of the fifth aspect of the present disclosure, in the treatment of phenylketonuria.

The eighth aspect of the present disclosure provides a method for treating phenylketonuria, comprising administering to a subject an effective amount of at least one of the polynucleotide molecules, the expression cassette, the expression vector, the viral particle and the pharmaceutical composition of the present disclosure.

By using the polynucleotide molecules, expression cassettes, expression vectors, viral particles and/or pharmaceutical compositions provided by the present disclosure, optimizing the coding gene of PAH so that it can express human PAH more effectively, effective, persistent and stable expression of human PAH in the liver at a relatively low dose is achieved, so that the concentration of phenylalanine in the subject's blood is maintained at a low level for a long time, thereby phenylketonuria is treated. Furthermore, the polynucleotide molecules, expression cassettes, expression vectors, viral particles and/or pharmaceutical compositions disclosed herein can be used at lower dosages and thus exhibit lower potential liver toxicity.

### The Description of Drawings

Fig. 1 is a schematic diagram illustrating the structure of a PAH expression cassette of a recombinant AAV (rAAV) vector of the present disclosure.

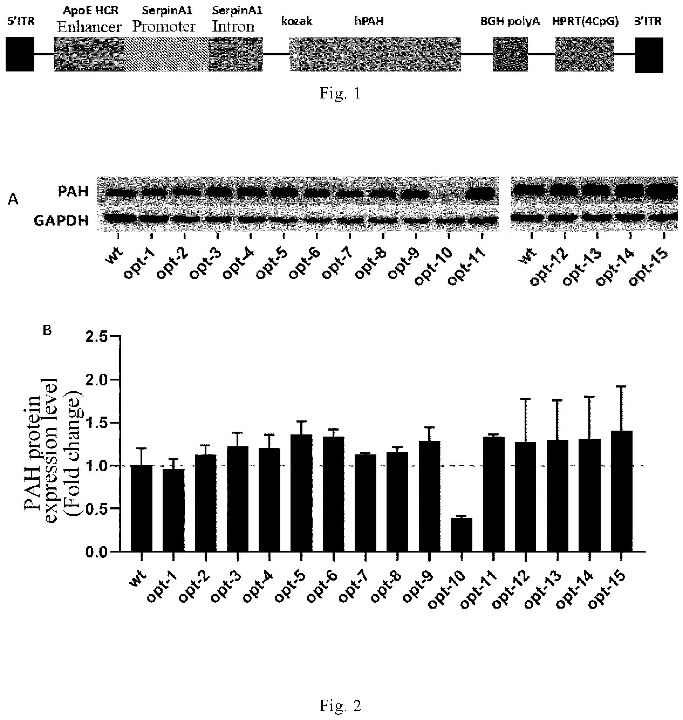

Fig. 2 illustrates the *in vitro* expression of a codon-optimized PAH opt gene in HepG2 cells transfected with plasmids. Wherein, Figure A is a representative image of Western blotting of PAH protein; Figure B shows the quantitative results of Western blotting of PAH protein to detect the expression levels of PAH and GAPDH in HepG2 cells (blank plasmid transfection as control), in which WT or opt gene was expressed using pAAV8-ATT-PAH WT or pAAV8-ATT-PAH opt plasmid, respectively. Cell lysates were harvested 2 days after transfection with equal amounts of plasmids. Equal amounts of total protein were separated by SDS-PAGE and then subjected to immunoblotting. The expression level of PAH was normalized with GAPDH and calculated as a ratio to the WT expression level.
Fig. 3A shows the effect of reducing Phe level in PKU model mice after hydrodynamic tail vein (HTV) injection of PAH WT and optimized genes by plasmids. At 0h, 6h, 24h, 3 days, 5 days, 10 days, 16 days and 19 days after injection, the blood of mice was collected to measure the Phe level in the blood; Fig. 3B illustrates the Phe level in the blood of PKU model mice at 10 days after injection.
Fig. 4 illustrates the effects of reducing Phe level in PKU model mice by AAV8-ATT-PAH WT and optimized gene viruses. During the 4 weeks after the injection, the blood of the mice was collected every week to measure the Phe level in the blood.
Fig. 5 is a schematic diagram of the structure of the PAH expression cassette of the recombinant AAV (rAAV) vector containing different promoters.
Fig. 6 illustrates the effect of reducing Phe level in PKU model mice after HTV injection of PAH WT expression cassettes containing different promoters via plasmids. On the 3rd day after the injection, the blood of the mice was collected to measure the Phe level in blood.
Fig. 7 is a schematic diagram of the structure of the PAH expression cassette of the recombinant AAV (rAAV) vector containing different expression regulatory elements.
Fig. 8 illustrates the effect of reducing Phe in PKU model mice after HTV injection of PAH expression cassettes containing different expression regulatory elements via plasmids. On the 3rd day after the injection, the bloods of the mice were collected to measure the Phe level in blood.
Fig. 9 is a schematic diagram of the structure of the PAH expression cassettes of the optimized recombinant AAV (rAAV) vectors containing different stuffer sequences.
Fig. 10A illustrates the effect of reducing Phe level in PKU model miceby AAV8-ATT-PAH opt9 viruses with different stuffer sequences. Blood was collected from mice every week for 8 weeks after injection to measure the Phe level in the blood. Fig. 10B illustrates the effect of reducing Phe level in PKU model mice by AAV8-ATT-PAH opt9 and AAV8-ATT-PAH opt-HPRT (4CpG) viruses. Within 4 weeks after injection, the blood of mice was collected every week to measure the Phe level in the blood.
Fig. 11 illustrates the functional test results of the expression product PAH of AAV8-ATT-PAH opt-HPRT (4CpG) virus in HepG2 cell lines to reduce the concentration of Phe. In HepG2 cells transiently transfected with AAVR, AAV8-ATT-PAH opt-HPRT (4CpG) virus was infected with MOIs of 0, 5E4 (i.e., 5 × 10⁴), 1E5, and 2E5, and the change in Phe concentration was calculated using the HepG2 cell line not infected with the virus as a reference.
Fig. 12A illustrates the effect of reducing Phe level in PKU model mice by different doses of AAV8-ATT-PAH opt9-HPRT (4CpG) virus, wherein the blood of the mice was collected weekly for 6 weeks after injection to measure the Phe level in the blood. Fig. 12B illustrates the effect of reducing Phe level in PKU model mice by different doses of AAV8-ATT-PAH opt9-HPRT (4CpG) virus. Within 6 weeks after injection, the blood of mice was collected weekly to measure the Phe level in the blood.

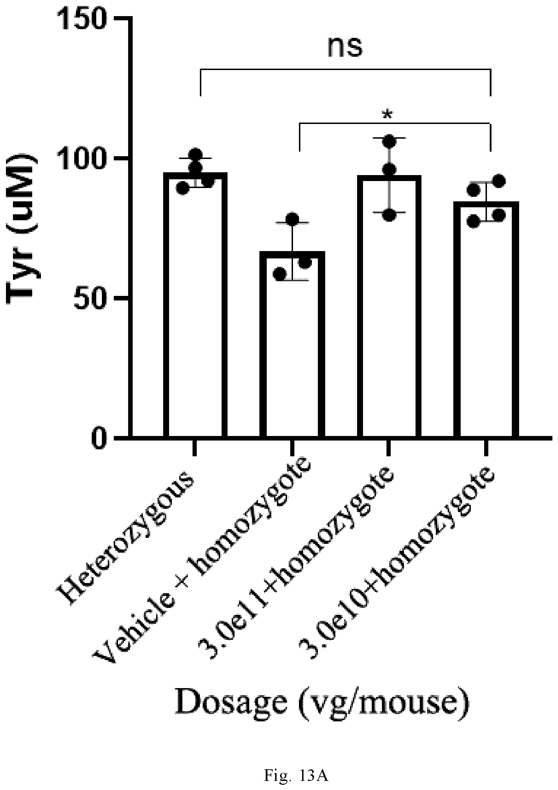

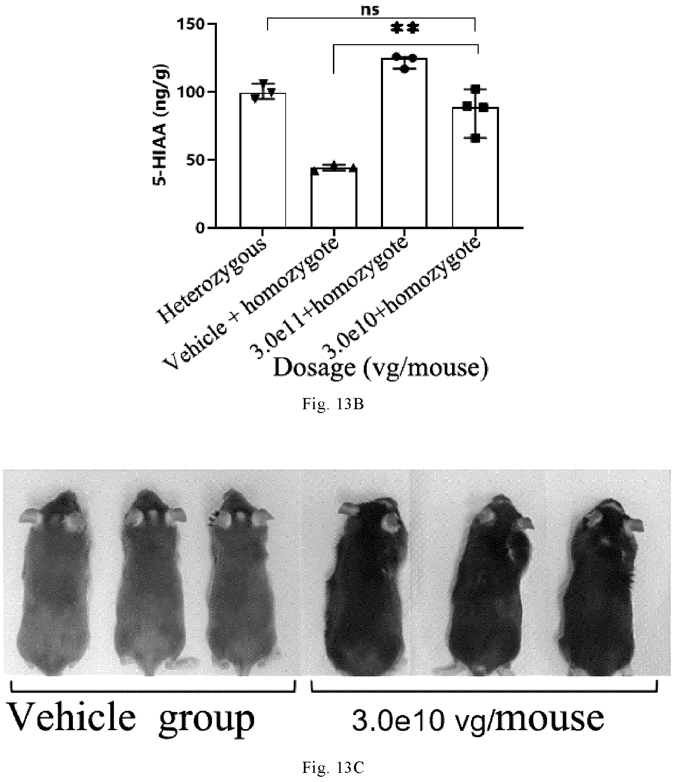

Fig. 13A illustrates the Tyr level in the brain tissue of PKU homozygous mice after 6 weeks of administration with AAV8-ATT-PAH opt9-HPRT (4CpG) virus at the dose of 3.0E10 and 3.0E11 vg/mouse, wherein heterozygous mice that were not administered with the drug served as a normal mouse control group, and homozygous mice that were administered with the vehicle served as an untreated control group. Fig. 13B illustrates the content of 5-hydroxyindoleacetic acid (5-HIAA) in mouse brain tissue. Fig. 13C illustrates the comparison of coat color between PKU homozygous mice and the vehicle group after administration at a dose of 3.0E10 vg/mouse for 3 weeks.

### Detailed Description of the Preferred Embodiment

In order to more clearly illustrate the embodiments of the present disclosure or the technical solutions in the prior art, the drawings required for use in the embodiments or the description of the prior art will be briefly introduced below. Obviously, the drawings described below are only one embodiment of the present disclosure, and for ordinary technicians in this field, other embodiments can also be obtained based on these drawings.

### Definition

In the present disclosure, unless otherwise defined, scientific and technical terms used herein have the meanings commonly understood by those skilled in the art. Furthermore, the terms and laboratory procedures related to protein and nucleic acid chemistry, molecular biology, cell and tissue culture, microbiology, and immunology used herein are terms and common procedures widely used in the corresponding fields. Meanwhile, for a better understanding of the present disclosure, definitions and explanations of relevant terms are provided below.

As used herein, the terms "a" and "an" and "the" and similar referents refer to both the singular and the plural, unless otherwise indicated herein or clearly contradicted by context.

As used herein, the terms "about", "substantially", and "similar to" mean within an acceptable error range for the particular value as determined by one of ordinary skill in the art, which error range may depend in part on the manner in which the value is measured or determined or on the limitations of the measurement system. As used herein, references herein to "about" a value or parameter include (and describe) embodiments directed to the value or parameter itself. For example, description referring to "about X" includes description of "X".

As used herein, "vector" refers to a recombinant plasmid or virus containing a nucleic acid to be delivered into a host cell *(in vitro* or *in vivo).*

As used herein, the term "polynucleotide molecule" or "nucleic acid" refers to a polymeric form of nucleotides of any length, either ribonucleotides or deoxyribonucleotides. Thus, the term includes, but is not limited to, single-, double-, or multi-stranded DNA or RNA, genomic DNA, cDNA, DNA-RNA hybrids, or polymers containing purine and pyrimidine bases or other natural, chemically or biochemically modified, non-natural or derived nucleotide bases. The backbone of a nucleic acid may comprise glycosides and phosphate groups (as typically found in RNA or DNA), or modified or substituted glycosides or phosphate groups. Alternatively, the backbone of the nucleic acid may comprises a polymer of synthetic subunits such as phosphoramidites and may thus be an oligodeoxynucleoside phosphoramidite (P-NH₂) or a mixed phosphoramidate-phosphodiester oligomer. In addition, a double-stranded nucleic acid can be obtained from a single-stranded polynucleotide product which is chemically synthesized (by synthesizing a complementary chain and annealing the chains under appropriate conditions, or by synthesizing a complementary chain *de novo* using a DNA polymerase with appropriate primers).

"Recombinant viral vector" refers to a recombinant polynucleotide vector that comprises one or more heterologous sequences (i.e., non-viral nucleic acid sequences). **In** the case of a recombinant AAV vector, the recombinant nucleic acid is flanked by at least one, and preferably two, inverted terminal repeats (ITRs).

"Recombinant AAV vector (rAAV vector)" refers to a polynucleotide vector comprising one or more heterologous sequences (i.e., nucleic acid sequences not derived from AAV) flanked by at least one, preferably two, AAV inverted terminal repeats (ITRs). The rAAV vector can replicate and be packaged into infectious viral particles when present in host cells that have been infected with an appropriate helper virus (or express appropriate helper functions) and that express the AAV rep and cap gene products (i.e., AAV Rep and Cap proteins). The rAAV vector may be referred to as a "pro-vector" when it is incorporated into a larger polynucleotide (e.g., in a chromosome or in another vector such as a plasmid used for cloning or transfection), which can be "rescued" via replication and encapsidation in the presence of AAV packaging functions and appropriate helper functions. The rAAV vector may be in any of a variety of forms, including but not limited to a plasmid, a linear artificial chromosome, which may be complexed with or encapsulated within a liposome, and, in embodiments, encapsulated within a viral particle, particularly an AAV particle. The rAAV vector can be packaged into an AAV viral capsid to generate a "recombinant adeno-associated virus particle (rAAV particle)". AAV helper functions (i.e., functions that allow AAV to be replicated and packaged by a host cell) can be provided in any of a variety of forms, including but not limited to helper viruses or helper viral genes that assist in AAV replication and packaging. Other AAV helper functions are known in the art.

"rAAV virus" or "rAAV viral particle" refers to a viral particle composed of at least one AAV capsid protein and an encapsidated rAAV vector genome.

"Heterologous" means derived from an entity that is genotypically different from the rest of the entity to which it is being compared or into which it is introduced or incorporated. For example, a nucleic acid that is introduced into a different cell type by genetic engineering techniques is a heterologous nucleic acid (and, when expressed, may encode a heterologous polypeptide). Similarly, a cellular sequence (e.g., a gene or portion thereof) incorporated into a viral vector is a heterologous nucleotide sequence relative to the vector.

As used in reference to viral titer, the term "genomic particles (gp)", "genome equivalents" or "genome copies" refers to the number of viral particles containing the recombinant AAV DNA genome, regardless of their infectiousness or functionality. The number of genomic particles in a particular vector preparation can be measured by methods as described in the Examples herein or, for example, in Clark et al. (1999) Hum. Gene Ther., 10:1031-1039; Veldwijk et al. (2002) Mol. Ther., 6:272-278.

As used in reference to viral titer, the term "infectious unit (iu)", "infectious particles" or "replication units" refers to the number of recombinant AAV vector particles that are infectious and replication competent as measured by the infectious center assay, also known as the replication center assay, as described, e.g., in McLaughlin et al. (1988) J. Virol., 62:1963-1973.

As used in reference to viral titer, the term "transduction unit (tu)" refers to the number of infectious recombinant AAV vector particles that result in the production of a functional transgene product, as measured in a functional assay, such as described in the Examples herein or, e.g., Xiao et al. (1997) Exp. Neurobiol., 144: 113-124; or Fisher et al. (1996) J. Virol., 70: 520-532 (LFU assay).

"Inverted terminal repeat" or "ITR" sequence is a term well known in the art and refers to relatively short sequences found at the ends of the viral genome, in opposite orientations.

"AAV inverted terminal repeat (ITR)" sequence is a term well known in the art, which is a sequence of about 145 nucleotides present at both ends of the native single-stranded AAV genome. The outermost 125 nucleotides of the ITR can be present in either of two alternative orientations, resulting in heterogeneity between different AAV genomes and between the two ends of a single AAV genome. The outermost 125 nucleotides also contain several shorter regions of self-complementarity (termed the A, A', B, B', C, C', and D regions) that allow interstrand base pairing to occur within this ITR portion.

A "helper virus" for AAV refers to a virus that allows AAV (which is a defective parvovirus) to replicate and be packaged in host cells. A number of such helper viruses have been identified, including adenovirus, herpes virus, and poxviruses such as vaccinia. Adenoviruses encompass several different subclasses, although subclass C adenovirus type 5 (Ad5) is the most commonly used. A variety of adenoviruses of human, non-human mammalian, and avian origin are known and available from depositories such as the ATCC. The herpes virus family, which is also available from depositories such as the ATCC, includes, for example, herpes simplex viruses (HSV), Epstein-Barr viruses (EBV), cytomegaloviruses (CMV), and pseudorabies viruses (PRV).

In the present disclosure, "stuffer sequence" refers to a human non-coding sequence added to make the nucleotide sequence length of the recombinant AAV expression cassette close to the length of the wild-type AAV genome. In some embodiments of the present disclosure, the addition of a stuffer sequence can enable the expression cassette or the polynucleotide sequence carrying it to have a higher viral packaging yield when expressed using an AAV viral vector.

"Percent (%) sequence identity" relative to a reference polypeptide or nucleic acid sequence is defined as the percentage of amino acid residues or nucleotides in a candidate sequence that are identical to the amino acid residues or nucleotides in the reference polypeptide or nucleic acid sequence, after aligning the sequences and introducing gaps, if necessary, to achieve the maximum percent sequence identity and not considering any conservative substitutions as part of the sequence identity. Alignment for purposes of determining percent amino acid or nucleic acid sequence identity can be achieved in various ways that are within the skill in the art, for instance, using publicly available computer software programs such as BLAST, BLAST-2, ALIGN or Megalign (DNASTAR) software. The preferred alignment software is ALIGN Plus (Scientific and Educational Software, Pennsylvania). Those skilled in the art can determine appropriate parameters for measuring alignment, including any algorithms needed to achieve maximal alignment over the full length of the sequences being compared. For purposes herein, the % amino acid sequence identity of a given amino acid sequence A to, with, or against a given amino acid sequence B (which may alternatively be referred to as having or comprising an amino acid sequence A having a certain % amino acid sequence identity to, with, or against a given amino acid sequence B) is calculated as follows: 100 multiplied by a fraction of X/Y, wherein X is the number of amino acid residues scored as identical matches by the sequence alignment program in the alignment of A and B, and wherein Y is the total number of amino acid residues in B. It will be appreciated that wherein the length of amino acid sequence A is not equal to the length of amino acid sequence B, the % amino acid sequence identity of A to B will not be equal to the % amino acid sequence identity of B to A. For purposes herein, the % nucleic acid sequence identity of a given nucleic acid sequence C to, with, or against a given nucleic acid sequence D (which may alternatively be referred to as a given nucleic acid sequence C having or comprising a certain % nucleic acid sequence identity to, with, or against a given nucleic acid sequence D) is calculated as follows: 100 multiplied by a fraction of W/Z, wherein W is the number of nucleotides scored as identical matches by a sequence alignment program in the alignment of C and D, and wherein Z is the total number of nucleotides in D. It will be appreciated that wherein the length of nucleic acid sequence C is not equal to the length of nucleic acid sequence D, the % nucleic acid sequence identity of C to D will not be equal to the % nucleic acid sequence identity of D to C.

An "effective amount" is an amount sufficient to affect beneficial or desired results, including clinical results (e.g., amelioration of symptoms, achievement of a clinical endpoint, etc.). An effective amount may be administered in one or more administrations. With respect to a disease state, an effective amount is an amount sufficient to ameliorate, stabilize or delay progression of the disease. For example, an effective amount of rAAV particles expresses a desired amount of a heterologous nucleic acid, such as a therapeutic polypeptide or therapeutic nucleic acid.

An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In some embodiments, the individual or subject is a human.

As used herein, "treatment" or "treating" is an approach for obtaining beneficial or desired clinical results. For purposes of the present disclosure, beneficial or desired clinical results include, but are not limited to, amelioration of symptoms, reduction in extent of disease, stabilized (e.g., non-worsening) state of disease, prevention of spread of disease (e.g., metastasis), delay or slowing of disease progression, improvement or palliation of the disease state, and remission (whether partial or complete), whether detectable or undetectable. "Treatment" or "treating" can also mean prolonging survival as compared to expected survival without receiving treatment.

The unit vg (Vector Genomes) represents the viral genome copy number.

The virus multiplicity of infection (MOI) means the ratio of the number of viruses to bacteria during infection, that is, the average number of phages that infect each bacterium.

In a first aspect, the present disclosure provides a polynucleotide molecule encoding a PAH protein, comprsing a nucleotide sequence having 90% or more identity with the nucleotide sequence set forth in SEQ ID NO. 10, SEQ ID NO. 11, SEQ ID NO. 12, SEQ ID NO. 13, SEQ ID NO. 14, SEQ ID NO. 15, SEQ ID NO. 16, SEQ ID NO. 17, SEQ ID NO. 19, SEQ ID NO. 20, SEQ ID NO. 21, SEQ ID NO. 22 or SEQ ID NO. 23; preferably a nucleotide sequence having 91%, 92%, 93%, 94%, 95%, 96%, 97%, 98%, 99% or more identity; more preferably a nucleotide sequence having 98% or more identity.

In some embodiments, the polynucleotide molecule has a nucleotide sequence set forth in SEQ ID NO. 10, SEQ ID NO. 11, SEQ ID NO. 12, SEQ ID NO. 13, SEQ ID NO. 14, SEQ ID NO. 15, SEQ ID NO. 16, SEQ ID NO. 17, SEQ ID NO. 19, SEQ ID NO. 20, SEQ ID NO. 21, SEQ ID NO. 22 or SEQ ID NO. 23.

In some embodiments, the codon-optimized human PAH protein encoding gene has a nucleotide sequence set forth in SEQ ID NO. 17.

The second aspect of the present disclosure provides an expression cassette, which comprises the polynucleotide molecule provided in the first aspect of the present disclosure, and a promoter operably linked to the polynucleotide molecule.

In some embodiments, the promoter is a specific or non-specific promoter.

In some embodiments, the promoter comprises a core promoter.

In some embodiments, the promoter may be a constitutive promoter; preferably, the constitutive promoter is selected from at least one of a CMV promoter, an EF1A promoter, an EFS promoter, a CAG promoter, a CBh promoter, an SFFV promoter, an MSCV promoter, an SV40 promoter, an mPGK promoter, an hPGK promoter, and a UBC promoter, etc.

In some embodiments, the promoter is an inducible promoter; preferably, the inducible promoter comprises at least one of a tetracycline-regulated promoter, an alcohol-regulated promoter, a steroid-regulated promoter, a metal-regulated promoter, a pathogenicity-regulated promoter, a temperature/heat-inducible promoter, a light-regulated promoter, and an IPTG-inducible promoter. In some embodiments, the tetracycline-regulated promoter is selected from a Tet on promoter, a Tet off promoter, and a Tet Activator promoter. In some embodiments, the alcohol-regulated promoter is selected from the alcohol dehydrogenase I (alcA) gene promoter and a promoter responsive to alcohol transactivator protein (AlcR). In some embodiments, the steroid-regulated promoter is selected from the group consisting of a rat glucocorticoid receptor promoter, a human estrogen receptor promoter, a moth ecdysone receptor promoter, a retinoid promoter, and a thyroid receptor superfamily promoter. In some embodiments, the metal-regulated promoter is selected from yeast, mouse and human metallothionein promoters. In some embodiments, the pathogenicity-regulated promoter is selected from a salicylic acid-regulated promoter, an ethylene-regulated promoter, and a benzothiadiazole-regulated (BTH) promoter. In some embodiments of the present disclosure, the temperature/heat-inducible promoter is selected from an HSP-70 promoter, an HSP-90 promoter, and a soybean heat shock promoter. In some embodiments of the present disclosure, the light-regulated promoter is a light-responsive promoter of plant cells.

In some preferred embodiments, the promoter is a liver-specific promoter; some non-limiting examples of liver-specific promoters include, but are not limited to, ApoA-I promoter, ApoA-II promoter, ApoA-IV promoter, ApoB promoter, ApoC-1 promoter, ApoC-II promoter, ApoC-III promoter, ApoE promoter, albumin promoter, alpha-fetoprotein promoter, phosphoenolpyruvate carboxykinase (PCK1) promoter, phosphoenolpyruvate carboxykinase 2 (PCK2) promoter, transthyretin (TTR) promoter, α-antitrypsin (AAT or SerpinA1) promoter, TK (thymidine kinase) promoter, hemoglobin promoter, alcohol dehydrogenase 6 promoter, cholesterol 7α-25 hydroxylase promoter, factor IX promoter, α-microglobulin promoter, SV40 promoter, CMV promoter, Rous sarcoma virus-LTR promoter, HBV promoter, ALB promoter and TBG promoter. Of course, the minimal promoters derived from these promoters can also be used. More preferably, the liver-specific promoter is human α1 antitrypsin promoter (hAAT or SERPINA1 promoter), preferably, the core promoter comprises a nucleotide sequence having 90% or more identity with the nucleotide sequence set forth in SEQ ID NO. 3, 24, 25, 30, 33 or 38, preferably a nucleotide sequence having 91%, 92%, 93%, 94%, 95%, 96%, 97%, 98%, 99% or more identity, more preferably a nucleotide sequence having 98%, 99% or more identity; preferably, the core promoter has a nucleotide sequence set forth in SEQ ID NO. 3, 24, 25, 30, 33 or 38; more preferably, more preferably, the nucleotide sequence of the core promoter is set forth in SEQ ID NO. 3.

In some embodiments, the expression cassette further comprises an expression control element, which is operably linked to the polynucleotide molecule.

In some embodiments, the expression control element is selected from at least one of a transcription/translation control signal, an enhancer, an intron, a polyA signal, an ITR, an insulator, an RNA processing signal, and an element that enhances the stability of mRNA and protein.

In some embodiments, the expression cassette comprises a 5'ITR; preferably, the 5'ITR comprises a nucleotide sequence having 90% or more identity with the nucleotide sequence set forth in SEQ ID NO. 1, preferably a nucleotide sequence having 91%, 92%, 93%, 94%, 95%, 96%, 97%, 98%, 99% or more identity, more preferably a nucleotide sequence having 98%, 99% or more identity; more preferably, the 5'ITR has the nucleotide sequence set forth in SEQ ID NO. 1.

In some embodiments, the expression cassette comprises a 3' ITR. In some preferred embodiments, the 3'ITR comprises a nucleotide sequence having 90% or more identity with the nucleotide sequence set forth in SEQ ID NO. 8, preferably a nucleotide sequence having 91%, 92%, 93%, 94%, 95%, 96%, 97%, 98%, 99% or more identity, and more preferably a nucleotide sequence having 98%, 99% or more identity; more preferably, the 3'ITR has the nucleotide sequence set forth in SEQ ID NO. 8.

In some embodiments, the expression cassette further comprises an enhancer. In some preferred embodiments, the enhancer is selected from ApoE HCR enhancer or an active fragment thereof, CRMSBS2 enhancer or an active fragment thereof, TTRm enhancer or an active fragment thereof, and CMV enhancer or an active fragment thereof; more preferably, the enhancer comprises a nucleotide sequence having 90% or more identity with the nucleotide sequence set forth in SEQ ID NO. 2, 29, 32, 37 or 40, preferably a nucleotide sequence having 91%, 92%, 93%, 94%, 95%, 96%, 97%, 98%, 99% or more identity, and more preferably a nucleotide sequence having 98%, 99% or more identity; preferably, the enhancer has a nucleotide sequence set forth in SEQ ID NO. 2, 29, 32, 37 or 40; more preferably, the nucleotide sequence of the enhancer is set forth in SEQ ID NO. 2.

In some embodiments, the expression cassette further comprises an intron; preferably, the intron is a truncated α1 antitrypsin intron or an active fragment thereof, a β-globin 2 intron or an active fragment thereof, an SV40 intron or an active fragment thereof, and an intron of a minute virus of mice or an active fragment thereof; preferably, the intron comprises a nucleotide sequence having 90% or more identity with the nucleotide sequence set forth in SEQ ID NO. 4, 26, 27, 28, 31 or 34, preferably a nucleotide sequence having 91%, 92%, 93%, 94%, 95%, 96%, 97%, 98%, 99% or more identity, and more preferably a nucleotide sequence having 98%, 99% or more identity; preferably, the intron has a nucleotide sequence set forth in SEQ ID NO. 4, 26, 27, 28, 31 or 34; more preferably, the nucleotide sequence of the intron is set forth in SEQ ID NO. 4.

In some embodiments, the promoter in the expression cassette is a combined promoter comprising an upstream regulatory element, a core promoter, and an intron.

In some embodiments, the upstream regulatory element is an enhancer or an active fragment thereof.

In some embodiments, the combined promoter comprises a nucleotide sequence having 90% or more identity with the nucleotide sequence set forth in SEQ ID NO. 44, 45, 46, 47, 48 or 49, preferably a nucleotide sequence having 91%, 92%, 93%, 94%, 95%, 96%, 97%, 98%, 99% or more identity, and more preferably a nucleotide sequence having 98%, 99% or more identity; preferably, the combined promoter has a nucleotide sequence set forth in SEQ ID NO. 44, 45, 46, 47, 48 or 49; more preferably, the nucleotide sequence of the combined promoter is set forth in SEQ ID NO. 44.

In some embodiments, the expression cassette further comprises a polyA signal; preferably, the polyA signal is at least one of bovine growth hormone poly A (BGH poly A), short poly A, SV40 polyA, and human β-globin poly A; preferably, the polyA signal comprises a nucleotide sequence having 90% or more identity with the nucleotide sequence set forth in SEQ ID NO. 7, preferably a nucleotide sequence having 91%, 92%, 93%, 94%, 95%, 96%, 97%, 98%, 99% or more identity, more preferably a nucleotide sequence having 98%, 99% or more identity; more preferably, the nucleotide sequence of the polyA signal is set forth in SEQ ID NO. 7.

In some embodiments, the expression cassette comprises an optimized stuffer sequence; preferably, the stuffer sequence is selected from a partial intron sequence of hypoxanthine phosphoribosyltransferase (HPRT) and a Woodchuck Hepatitis Virus Posttranscriptional Regulatory Element (WPRE); preferably, the number of CpG sequences contained in the partial intron sequence does not exceed 100, 80, 60, 50, 40, 30, 20, 15, 10, 9, 8, 7, 6, 5, 4, 3, 2 or 1; preferably, the partial intron sequence does not contain a CpG sequence or a Woodchuck Hepatitis Virus Posttranscriptional Regulatory Element (WPRE); preferably, the stuffer sequence is a partial intron sequence of hypoxanthine phosphoribosyltransferase (HPRT); preferably, the stuffer sequence comprises a nucleotide sequence having 90% or more identity with the nucleotide sequence set forth in SEQ ID NO. 39 or SEQ ID NO. 43, preferably a nucleotide sequence having 91%, 92%, 93%, 94%, 95%, 96%, 97%, 98%, 99% or more identity, and more preferably a nucleotide sequence having 98%, 99% or more identity; more preferably, the nucleotide sequence of the filler sequence is set forth in SEQ ID NO. 39 or SEQ ID NO. 43.

In some embodiments, the expression cassette includes a Kozak start sequence; the Kozak start sequence comprises a nucleotide sequence having 90% or more identity with the nucleotide sequence set forth in SEQ ID NO. 5, preferably a nucleotide sequence having 91%, 92%, 93%, 94%, 95%, 96%, 97%, 98%, 99% or more identity, and more preferably a nucleotide sequence having 98%, 99% or more identity; more preferably, the Kozak start sequence has the nucleotide sequence set forth in SEQ ID NO. 5.

In some embodiments, the expression cassette comprises a 5'ITR, an ApoE HCR enhancer, a human α1 antitrypsin promoter (SERPINA1 promoter), a truncated α1 antitrypsin intron (SerpinA1 intron), a Kozak start sequence (GCCACC, SEQ ID NO. 5), the polynucleotide molecule, a BGH poly A, a partial intron sequence of hypoxanthine phosphoribosyltransferase (HPRT) (HPRT (4CpG)) and a 3'ITR; preferably, the expression cassette comprises a nucleotide sequence having 90% or more identity with the nucleotide sequence set forth in SEQ ID NO. 80, SEQ ID NO. 81, SEQ ID NO. 82, SEQ ID NO. 83, SEQ ID NO. 84, SEQ ID NO. 85, SEQ ID NO. 86, SEQ ID NO. 87, SEQ ID NO. 89, SEQ ID NO. 90, SEQ ID NO. 91, SEQ ID NO. 92 or SEQ ID NO. 93, preferably a nucleotide sequence having 91%, 92%, 93%, 94%, 95%, 96%, 97%, 98%, 99% or more identity, and more preferably a nucleotide sequence having 98%, 99% or more identity; more preferably, the expression cassette has a nucleotide sequence set forth in SEQ ID NO.80, SEQ ID NO. 81, SEQ ID NO. 82, SEQ ID NO. 83, SEQ ID NO. 84, SEQ ID NO. 85, SEQ ID NO. 86, SEQ ID NO. 87, SEQ ID NO. 89, SEQ ID NO. 90, SEQ ID NO. 91, SEQ ID NO. 92 or SEQ ID NO. 93.

The third aspect of the present disclosure provides an expression vector, which comprises the polynucleotide molecule provided by the first aspect of the present disclosure or the expression cassette provided by the second aspect of the present disclosure.

In some embodiments, the expression vector further comprises a gene encoding a marker, preferably, the marker is selected from at least one of an antibiotic resistance protein, a toxin resistance protein, a colored or fluorescent or luminescent protein, and a protein that mediates enhanced cell growth and/or gene amplification.

In some embodiments, the antibiotic is selected from at least one of ampicillin, neomycin, G418, puromycin and blasticidin.

In some embodiments, the toxin is selected from at least one of anthrax toxin and diphtheria toxin.

In some embodiments, the colored or fluorescent or luminescent protein is selected from at least one of green fluorescent protein, enhanced green fluorescent protein, red fluorescent protein and luciferase.

In some embodiments, the protein that mediates enhanced cell growth and/or gene amplification is dihydrofolate reductase (DHFR).

In some embodiments, the expression vector comprises an origin of replication; preferably, the origin of replication sequence is selected from at least one of f1 bacteriophage ori, RK2oriV, pUC ori and pSC101ori.

In some embodiments, the expression vector is selected from a plasmid, a cosmid, a viral vector, an RNA vector, or a linear or circular DNA or RNA molecule.

In some embodiments, the plasmid is selected from pCI, puc57, pcDNA3, pSG5, pJ603 or pCMV.

In some embodiments, the viral vector is selected from a retrovirus, an adenovirus, a parvovirus (e.g., an adeno-associated virus), a coronavirus, a negative-strand RNA virus such as an orthomyxovirus (e.g., influenza virus), a rhabdovirus (e.g., rabies and vesicular stomatitis virus), a paramyxovirus (e.g., mammary gland and Sendai), a positive-strand RNA virus (such as a picornavirus and an alphavirus), or a double-stranded DNA virus; the double-stranded DBA virus is selected from an adenovirus, a herpesvirus (e.g., herpes simplex virus type 1 and 2, Epstein-Barr virus, cytomegalovirus), a poxvirus (e.g., vaccinia virus, fowlpox virus, and canarypox virus), a Norwalk virus, a togavirus, a flavivirus, a reovirus, a papovavirus, a hepadnavirus, a baculovirus, or a hepatitis virus.

In some embodiments, the retrovirus is selected from avian leukocytoproliferative-sarcoma, mammalian C-type, B-type virus, D-type virus, HTLV-BLV collection, lentivirus or foamy virus.

In some embodiments, the lentiviral vector is selected from HIV-1, HIV-2, SIV, FIV, BIV, EIAV, CAEV, or ovine demyelinating leukoencephalitis lentivirus.

In some embodiments, the expression vector is an adeno-associated virus (AAV) vector.

In some embodiments, the adeno-associated virus is selected from AAV type 1, AAV type 2, AAV type 3, AAV type 4, AAV type 5, AAV type 6, AAV type 7, AAV type 8, AAV type 9, AAV type 10, avian AAV, bovine AAV, canine AAV, equine AAV, or ovine AAV.

In some embodiments, the expression cassette can be packaged in a rAAV vector with a capsid from any AAV serotype or a hybrid or variant thereof.

In some embodiments, the expression vector comprises a nucleotide sequence having 90% or more identity with the nucleotide sequence set forth in SEQ ID NO. 53, SEQ ID NO. 54, SEQ ID NO. 55, SEQ ID NO. 56, SEQ ID NO. 57, SEQ ID NO. 58, SEQ ID NO. 59, SEQ ID NO. 60, SEQ ID NO. 62, SEQ ID NO. 63, SEQ ID NO. 64, SEQ ID NO. 65, SEQ ID NO. 66, SEQ ID NO. 72, SEQ ID NO. 73, SEQ ID NO. 74, SEQ ID NO. 75, SEQ ID NO. 76, SEQ ID NO. 77, SEQ ID NO. 78, SEQ ID NO. 96, SEQ ID NO. 97, SEQ ID NO. 98, SEQ ID NO. 99, SEQ ID NO. 100, SEQ ID NO. 101, SEQ ID NO. 102, SEQ ID NO. 103, SEQ ID NO. 105, SEQ ID NO. 106, SEQ ID NO. 107, SEQ ID NO. 108 or SEQ ID NO. 109, preferably a nucleotide sequence with 91%, 92%, 93%, 94%, 95%, 96%, 97%, 98%, 99% or more identity, and more preferably a nucleotide sequence with 98%, 99% or more identity; more preferably, the expression vector has a nucleotide sequence set forth in SEQ ID NO. 53, SEQ ID NO. 54, SEQ ID NO. 55, SEQ ID NO. 56, SEQ ID NO. 57, SEQ ID NO. 58, SEQ ID NO. 59, SEQ ID NO. 60, SEQ ID NO. 62, SEQ ID NO. 63, SEQ ID NO. 64, SEQ ID NO. 65, SEQ ID NO. 66, SEQ ID NO. 72, SEQ ID NO. 73, SEQ ID NO. 74, SEQ ID NO. 75, SEQ ID NO. 76, SEQ ID NO. 77, SEQ ID NO. 78, SEQ ID NO. 96, SEQ ID NO. 97, SEQ ID NO. 98, SEQ ID NO. 99, SEQ ID NO. 100, SEQ ID NO. 101, SEQ ID NO. 102, SEQ ID NO. 103, SEQ ID NO. 105, SEQ ID NO. 106, SEQ ID NO. 107, SEQ ID NO. 108 or SEQ ID NO. 109; more preferably, the expression vector has a nucleotide sequence set forth in SEQ ID NO. 76, SEQ ID NO. 73, SEQ ID NO. 60 or SEQ ID NO. 78.

In some embodiments, the expression vector has a nucleotide sequence set forth in SEQ ID NO. 73.

The fourth aspect of the present disclosure provides a virus particle, which comprises at least one of the polynucleotide molecule provided by the first aspect of the present disclosure, the expression cassette provided by the second aspect of the present disclosure, and the expression vector provided by the third aspect of the present disclosure.

The fifth aspect of the present disclosure provides a pharmaceutical composition for treating phenylketonuria, which comprises at least one of the polynucleotide molecule provided in the first aspect of the present disclosure, the expression cassette provided in the second aspect of the present disclosure, the expression vector provided in the third aspect of the present disclosure, and the virus particle provided in the fourth aspect of the present disclosure.

The sixth aspect of the present disclosure provides the use of the polynucleotide molecule provided in the first aspect of the present disclosure, the expression cassette provided in the second aspect of the present disclosure, the expression vector provided in the third aspect of the present disclosure, the virus particle provided in the fourth aspect of the present disclosure, or the pharmaceutical composition of the fifth aspect of the present disclosure, in the preparation of a medicament for treating phenylketonuria.

The seventh aspect of the present disclosure provides the use of the polynucleotide molecule provided in the first aspect of the present disclosure, the expression cassette provided in the second aspect of the present disclosure, the expression vector provided in the third aspect of the present disclosure, the viral particle mentioned in the fourth aspect of the present disclosure, or the pharmaceutical composition of the fifth aspect of the present disclosure, in the treatment of phenylketonuria.

The eighth aspect of the present disclosure provides a method for treating phenylketonuria, comprising administering to a subject an effective amount of at least one of the polynucleotide molecule, the expression cassette, the expression vector, the viral particle and the pharmaceutical composition of the present disclosure.

For purposes of clarity and concise description, features are described herein as part of the same or separate embodiments, however, it will be understood that the scope of the present disclosure may include embodiments having a combination of all or some of the described features.

Hereinafter, the present disclosure will be described in more detail with reference to specific examples; however, the examples are only for illustrative purposes and have no limiting effect on the present disclosure.

### Materials and methods:

### PAH expression cassette and AAV vector

All wild-type and codon-optimized PAH gene sequences used in this study were synthesized by GenScript. Codon optimization was performed using a modified version of the GenSmart codonoptimization tool. The AAV vector-mediated PAH expression cassette is shown in Fig. 1.

The entire initial AAV shuttle plasmid vector was synthesized by Universal Genetics according to the sequence of SEQ ID NO. 112. Wild-type AAV2 ITR sequence was recombined between BamHI and AleI restriction sites in the vector to repair the mutated ITR sequence in the vector. The Amp resistance gene between the ApaLI restriction sites of the initial shuttle plasmid vector was replaced with Kan, then it was recombined in between the HindIII and NheI restriction sites of the vector to increase the length of the vector to facilitate the packaging of the AAV virus. The CAG promoter sequence was amplified by PCR from the pCAGGS vector (GeneWiz) and recombined in between the SpeI and KpnI restriction sites of the shuttle plasmid vector to obtain the final shuttle plasmid vector (SEQ NO ID. 113). The CAG promoter comprises a CMV enhancer (SEQ ID NO. 40), a chicken β-actin promoter (SEQ ID NO. 41) and a chimeric intron (SEQ ID NO. 42).

All expression cassette sequences were cloned in between the SalI and BamHI restriction sites of the shuttle plasmid vector to obtain the shuttle plasmid comprising the PAH gene of interest whose expression is mediated by AAV vector.

### Methods for AAV vector production and purification

The production of AAV vector was conducted by a three-plasmid system, namely, a shuttle plasmid containing the PAH gene of interest, a pRepCap plasmid with the AAV vector repcap gene, and a helper plasmid Phelper (pRepCap plasmid, synthesized by GeneWiz according to the sequence of SEQ ID NO. 110, and a helper plasmid pHelper, synthesized by GeneWiz according to the sequence of SEQ NO ID. 111). By using PEI as a transfection reagent, the plasmids were co-transfected into HEK293 cells and the AAV viral vectors were packaged recombinantly. The cells were harvested 48-72 hours after transfection, and the harvested fluid was purified to obtain a recombinant AAV virus vector with a certain purity. The purification method was as follows:
First, the harvested fluid was pre-treated: HEK293 cells were fully lysed to release the AAV viral vector in the cells. Nuclease was added to digest the free nucleic acid. After digestion, deep filtration was used to remove large molecular impurities and cell fragments. After deep filtration, the filtrate was filtered twice to obtain a clarified fluid for affinity loading.

Utilizing the specific adsorption of ligands to proteins, the AAV viral vectors in the harvest fluid were captured via affinity chromatography, and most process-related impurities were removed, achieving the purposes of concentration and impurity removal. The collected eluate was mixed, neutralized with neutralization buffer, and stored in a sterile storage bottle, ready for loading onto anion chromatography.

Full AAV particles were separated with empty AAV particles by utilizing the difference in isoelectric points of different components via anion chromatography, where residual impurities were further removed. The eluate was collected into a new sterile storage bottle, and then the buffer was replaced, by ultrafiltration, with the one that is stable for the preparation. At the same time, the virus was concentrated to a titer of about 1×10¹³ vg/mL. The AAV vectors were finally sterilized, filtered, and packaged for later use.

### Quantification of AAV vector titers

After the purification of AAV, the content of virus should be measured. The genome titer is the most classic test item to characterize the physical titer of AAV. The most common method for detecting genome titer is to design primer probes targeting the rAAV genome sequence and then perform Q-PCR detection.

Given that the ORF coding frame has been codon-optimized in the present disclosure, and the screening of multiple vector structures was involved, the common sequence in the vectors, which was PolyA, was selected as target for primer probes design, in order to ensure the quantitative stability and accuracy between different vector structures. F primer sequence: 5'-CAAGCCCATGTACACACCAG-3' (SEQ ID NO.114), R primer sequence: 5'-GGGCAAAGCTTCTGTCTGAG-3' (SEQ ID NO.115), probe sequence: 5'-CTGACATCTGCCACGAGCTGCTGGGCCA-3' (SEQ ID NO.116).

In the process of genome titer detection, the standard curve should be established first. The positive standard plasmid was diluted to 2×10⁷, 2×10⁶, 2×10⁵, 2×10⁴, 2×10³, and 2×10² copies/µl respectively, which were used as a standard curve template. The linearity and amplification efficiency of the standard curve need to be controlled. Generally, R² >0.99 and an amplification efficiency between 90% and 110% were required. The pre-treated rAAV sample was then diluted and was subjected to QPCR detection. The Ct value of the sample should be within the range of the standard curve. The Ct value of the sample was substituted into the standard curve to calculate the rAAV sample genome titer and identify the product content.

### In vitro cell plasmid transfection experiment

Human hepatoma cell HepG2 cells were digested and seeded into 96-well plates at 2.5×10⁴ cells/well. At the same time, Lipo3K/DNA transfection complex was added for plasmid transfection experiment. First, Lipofectamine 3000 and P3000 (Thermo, L3000015) transfection reagents were premixed with the plasmid, added to the HepG2 cells in a 96-well plate, with 60ng /well of PAH-opt/WT expression plasmid, 90nL /well of P3000 and 90nL /well of Lipo 3000 (Thermo, L3000015), and cultured in a CO₂ constant-temperature incubator for 48 h.

### Western blot analysis

60 µL of RIPA lysis buffer (Beyotime, P0013B) containing 1× SDS loading buffer was added to each well of a 96-well plate inoculated with cultured cells. The cells were shaken and lysed for 10 min, then denatured at 95°C for 10 min. 10 µL of protein was loaded for SDS-PAGE electrophoresis and then transferred to a PVDF membrane, incubated with Anti-PAH (SantaCruz, sc-271258) and Anti-GAPDH (TransGen, HC301) antibodies, and imaged and analyzed using the ChemiDoc Touch Imaging System (Bio-Rad).

### Hydrodynamic tail vein injection of Plasmid into mice

A mouse was put in a suitable container, placed under an infrared lamp with a turned-on switch, and irradiated for several minutes. Then the mouse was taken out, and wiped the tail with an alcohol cotton ball to fully dilate the tail vein. The mouse was placed in a mouse holder with the tail exposed. A suitable syringe and needle were used to draw the sample to be injected, with a 1mL syringe needle and a 5mL syringe barrel. 2 mL (0.1 ml/g × mouse weight) of the sample to be injected was drawn and injected into the mouse through the tail vein. The injection should be completed within 5-8 seconds. The injection should be performed quickly and at a uniform speed. After the injection, the injection site was pressed with a dry cotton ball to stop the bleeding.

### Injection of virus samples into mice through tail vein

A mouse was put in a suitable container, placed under an infrared lamp with a turned-on switch, irradiated for several minutes. Then the mouse was taken out, and wiped the tail with an alcohol cotton ball to fully dilate the tail vein. The mouse was placed in a mouse holder with the tail exposed. A suitable syringe and needle were used to draw the sample to be injected, with a 1mL syringe needle and a 1mL syringe barrel. After diluting the virus sample according to the virus administration dosage, 200 µL of the diluted sample to be injected was drawn and injected into the mouse through tail vein. The injection should be completed in more than 10 seconds. The injection should be performed slowly and at a uniform speed. After the injection, the injection site was pressed with a dry cotton ball to stop the bleeding.

### Quantitative analysis of Phe in blood

Blood was collected from the periorbital area of the mouse. 20 µL of blood was dropped onto the blood collection card and air dried naturally. The concentration of phenylalanine in blood was quantified using a phenylalanine assay kit (FENGHUA, AN302) and calibrated with a standard blood card.

### In vitro test for the Phe-reducing effect of PAH

The cells to be tested were cultured on a 24-well plate. During the test, the cells to be tested were collected and rinsed once with PBS. 150 µL of reaction solution (containing 0.25% NP-40, 50 mM Hepes, 150 mM KCL, 800 mM L-Phe, 100 µg/mL Catalase, 400 µM FeNH₄ (SO₄)₂, 400 µM BH₄, 2 mM DTT) was added to each well and incubated at 37°C for 3 hours. 15 µL of the reaction solution was taken and dropped onto the blood collection card until air dried. The phenylalanine concentration in the blood was quantified using a phenylalanine assay kit (FENGHUA, AN302), and the reduction of Phe concentration was calculated.

### 5-HIAA Assay

After the mice were sacrificed, brain tissues were obtained. The level of 5-HIAA was detected by mass spectrometry by Suzhou PANOMIX Biomedical Tech Co., LTD. The specific method was as follows: the standard curve solution was prepared by gradient dilution of the 5-hydroxyindoleacetic acid standard. The brains tissue of the sample to be tested was homogenized, with the volume ratio of 1:1:10 = homogenate solution: internal standard working solution: methanol. The mixture was centrifuged at 12000rpm, 4°C for 10 minutes, and the supernatant was collected for testing. XDB-C18 analytical 4.6*150mm 5-Micron column and an electrospray ionization source were used for scanning detection using multiple reaction monitoring (MRM). The level of 5-HIAA in the sample to be tested was calculated according to the standard curve quantitative method.

### Quantitative analysis of Tyr in blood

Blood was collected from the periorbital area of mice. Serum was centrifuged using a 3 kDa ultrafiltration tube at 12000 xg for 20 min, and the filtrate was collected. The standard curve solution was prepared by gradient dilution of Tyr standard. The serum samples were centrifuged with 3 kDa ultrafiltration tubes at 12000xg for 20 min, and the filtrate was collected for testing. The signal was detected at the wavelength of 210 nm by using a Chromcore 120 C18 column. The level of Tyr in the sample to be tested was calculated according to the standard curve quantitative method.

### Example 1: Construction and purification of adeno-associated virus vector

### 1.1 Construction of recombinant adeno-associated virus vector

The structure of the PAH expression cassette is shown in Fig. 1. The PAH expression cassette comprises, from the 5' end to the 3' end, a 5'ITR, an ApoE HCR enhancer, a SerpinA1 promoter, a truncated SerpinA1 intron, a Kozak sequence, a target gene (wild-type human PAH gene (hPAH WT) or optimized PAH gene (hPAH opt)), a BGH polyA, a HPRT (4CpG) stuffer sequence and a 3'ITR. The nucleotide sequences of the PAH expression cassettes comprising different target genes are set forth in SEQ ID NOs. 79-93, respectively, wherein,
the nucleotide sequence of 5'ITR is set forth in SEQ ID NO. 1.

The ApoE HCR enhancer is the hepatocyte control region of human apolipoprotein E, and its nucleotide sequence is set forth in SEQ ID NO. 2.

The SerpinA1 promoter is human α1 antitrypsin promoter, and its nucleotide sequence is set forth in SEQ ID NO. 3.

The SerpinA1 intron is a truncated α1 antitrypsin intron with a length of 261 bp, and its nucleotide sequence is set forth in SEQ ID NO. 4.

The Kozak sequence is inserted before the PAH gene sequence, and its sequence is set forth in SEQ ID NO. 5.

The hPAH WT gene is derived from the wild-type human PAH gene (GeneID: 5053), and its gene sequence is set forth in SEQ ID NO. 6. The NCBI accession number of the wild-type hPAH WT protein it encodes is NP_000268.1.

The optimized PAH gene hPAH opt is a codon-optimized gene opt1-15 encoding a wild-type human PAH protein, and its nucleotide sequences are SEQ ID NOs. 9-23, respectively.

The BGH polyA is the bovine growth hormone polyadenylation signal, and its nucleotide sequence is set forth in SEQ ID NO. 7.

The HPRT (4CpG) stuffer sequence is a partial intron sequence of hypoxanthine phosphoribosyltransferase, and its nucleotide sequence is set forth in SEQ ID NO. 43.

The nucleotide sequence of 3'ITR is set forth in SEQ ID NO. 8.

The expression cassette comprising the wild-type human PAH gene (hPAH WT) was constructed into the shuttle plasmid as pAAV8-ATT-PAH-WT-HPRT. Wherein, SEQ ID NO. 51 showed the sequence of the shuttle plasmid that comprised an expression cassette comprising a wild-type human PAH gene (hPAH WT).

The expression cassettes comprising the optimized PAH genes opt 1~15 respectively were constructed as the shuttle plasmid pAAV8-ATT-PAH-opt1~15-HPRT, respectively. Wherein, SEQ ID NOs. 52 to 66 showed the sequences of shuttle plasmids that comprised expression cassettes comprising codon-optimized genes opt1~15, respectively.

### 1.2 Isolation and purification of adenoviral vectors

The AAV vectors were generated by using three-plasmid system. The shuttle plasmids pAAV8-ATT-PAH-WT-HPRT or pAAV8-ATT-PAH-opt1~15-HPRT comprising the PAH target genes respectively, the pRepCap plasmid with the AAV vector repcap gene, and the helper plasmid pHelper were used to co-transfect HEK293 cells, by using PEI as the transfection reagent. AAV viral vectors were recombinantly packaged, named as AAV8-ATT-PAH-WT-HPRT and AAV8-ATT-PAH-opt1~15-HPRT, respectively. The cells were harvested 48-72 hours after transfection, and the harvested fluid was purified by affinity chromatography, further purified by anion chromatography, concentrated by ultrafiltration, and exchanged for buffer. The genome titers of the purified recombinant AAV viral vectors were measured, and the vector were sterilized, filtered, and packaged for later use.

### Example 2: In vitro detection for expression of codon-optimized PAH opt

In this example, the in vitro expression level of codon-optimized PAH opt was evaluated in HepG2 cell line (purchased from the Cell Bank of Type Culture Collection Committee of the Chinese Academy of Sciences, catalog number: TCHu72). A shuttle plasmid pAAV8-ATT-PAH WT comprising the wild-type human PAH gene (hPAH WT) driven by ATT promoter was constructed, whose nucleotide sequence was set forth in SEQ ID NO. 94. Shuttle plasmids pAAV8-ATT-PAH opt 1~15 comprising the optimized PAH genes (hPAH opt1-15) respectively, whose nucleotide sequence were set forth in SEQ ID NO. 95~109 respectively, were also constructed. The genes of interest were introduced into cells by using the method in aforementioned *"in vitro* cell plasmid transfection experiment".

HepG2 cells were transiently transfected with plasmid pAAV8-ATT-PAH WT expressing PAH WT or plasmid pAAV8-ATT-PAH opt 1~15 expressing PAH opt, respectively. The expression of protein in cell lysates was evaluated by Western blot analysis, the results of which were shown in Fig. 2. Wherein, Figure A shows a representative image of Western blotting for PAH protein. Figure B shows the quantitative results. The expression levels of codon-optimized PAH genes opt2~9 and opt11-15 in HepG2 cells were significantly higher than that of the wild-type human PAH gene (WT), indicating that the codon-optimized PAH genes disclosed in the present invention have a higher expression level.

### Example 3: In vivo efficacy evaluation for codon-optimized PAH opt in a PKU mouse model

### 3.1 Comparison of codon-optimized plasmids for their functional activity in reducing Phe level in PKU mice

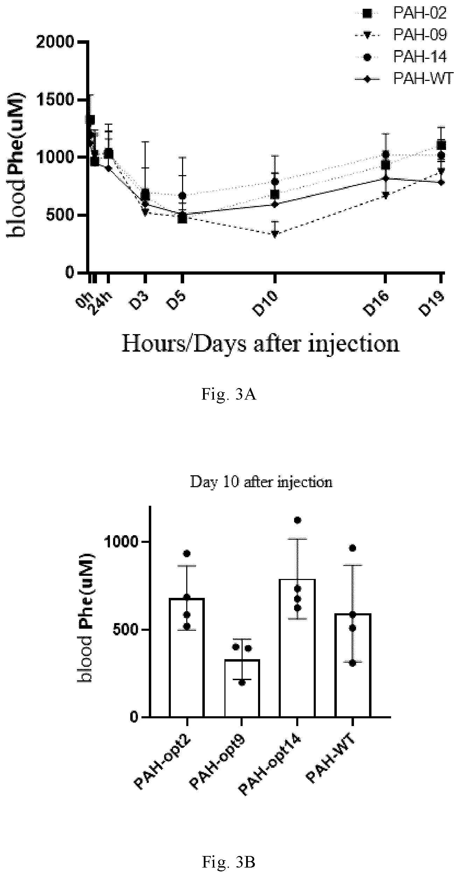

By hydrodynamic tail vein injection, PKU model mice (purchased from Beijing Chengtian Biotechnology Co., Ltd., strain BTBR-Pah<enu2> /J) were injected with 40µg of pAAV8-ATT-PAH WT or pAAV8-ATT-PAH opt2, opt9, opt14 plasmids. At 0 h, 6 h, 24 h, 3 days, 5 days, 10 days, 16 days, and 19 days after injection, the blood of mice was collected to determine the Phe level in the blood, and the effect of the PAH opt plasmid in reducing Phe was compared and analyzed. The results are shown in Figs. 3A and 3B. As shown in Fig. 3A, during D3~D16, blood Phe level of mice injected with PAH opt9 plasmid was lower than that of PAH WT, while the Phe levels of mice injected with PAH opt2 and mice injected with PAH opt14 plasmids were both higher than that of PAH WT. Since the plasmid will be metabolically degraded in the cell, the effect of reducing Phe level will be gradually weaken over time. As shown in Fig. 3B, on the 10th day after injection, the blood Phe level of mice injected with PAH opt9 plasmid was lower than that of PAH WT, PAH opt2 and PAH opt14. Therefore, it was demonstrated that the codon-optimized PAH opt9 exhibits a better effect in reducing Phe level.

### 3.2 Comparison of codon-optimized AAV viruses for their functional activity in reducing Phe in PKU mice

AAV viral vectors were recombinantly packaged with the shuttle plasmids of Example 2 by using the same method as 1.2 of Example 1, which were named as AAV8-ATT-PAH WT and AAV8-ATT-PAH opt2, 9, 11, and 14, respectively.

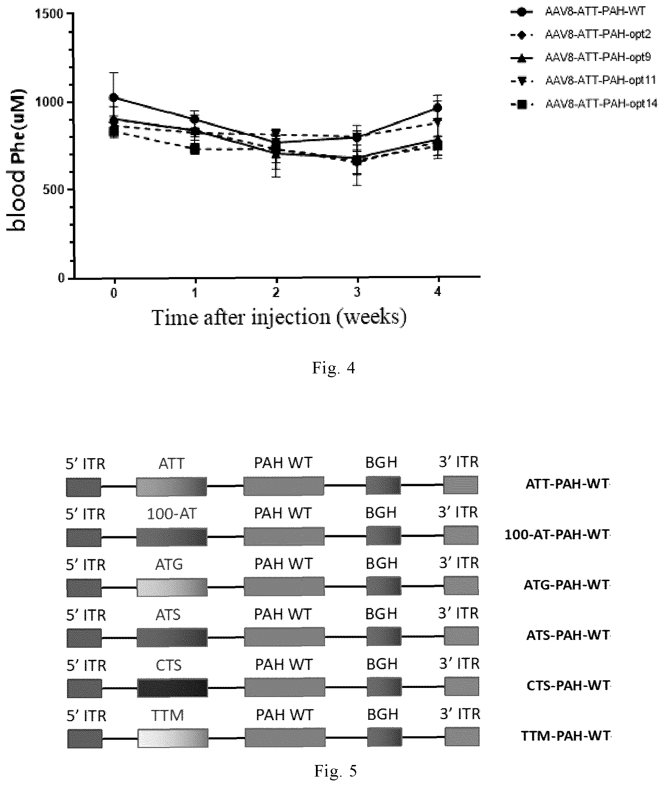

PKU model mice were injected intravenously with AAV8-ATT-PAH WT or AAV8-ATT-PAH opt2, opt9, opt11, or opt14 viruses at a dose of 1E10 vg/mouse. The blood of mice was collected every week after injection. Phe level in the blood was measured for 4 weeks. The effects of AAV8-ATT-PAH opt viruses in reducing Phe were compared and analyzed, which were shown in Fig. 4. As shown in Fig. 4, the blood Phe level of model mice injected with any one of AAV8-ATT-PAH opt2, 9, or 14 viruses was lower than that of AAV8-ATT-PAH WT, indicating that any of the codon-optimized AAV8-ATT-PAH opt2, 9, or 14 viruses has a better effect in reducing Phe level.

### Example 4: Screening for liver-specific promoter combinations in PKU mouse model

In this example, the effect of different promoters in driving PAH to reduce Phe level was evaluated in PKU model mice. The elements of the promoters for combination are shown in Table 1. ApoE HCR enhancer is a hepatocyte control region of human apolipoprotein E, whose nucleotide sequence is set forth in SEQ ID NO. 2. Core ApoE HCR enhancer is a hepatocyte control region of human apolipoprotein E, whose nucleotide sequence is set forth in SEQ ID NO. 37. CRMSBS2 enhancer is a modified Serpin1 enhancer, whose nucleotide sequence is set forth in SEQ ID NO. 29. TTRm enhancer is a mutated transthyretin enhancer region, whose nucleotide sequence is set forth in SEQ ID NO. 32. SerpinA1 promoter is human α1 antitrypsin promoter, whose nucleotide sequence is set forth in SEQ ID NO. 3. Core SerpinA1 promoter (218bp) is the core region of human α1 antitrypsin promoter, whose nucleotide sequence consists of SEQ ID NOs. 24 and 25. Core SerpinA1 promoter (254bp) is the core region of human α1 antitrypsin promoter, whose nucleotide sequence is set forth in SEQ ID NO.38. TTRm promoter (223 bp) is a mutant transthyretin promoter, whose nucleotide sequence is set forth in SEQ ID NO. 30. TTRm promoter (228 bp) is a mutant transthyretin promoter, whose nucleotide sequence is set forth in SEQ ID NO. 33. Truncated SerpinA1 intron (261 bp) is a truncated α1 antitrypsin intron, whose nucleotide sequence is set forth in SEQ ID NO. 4. Truncated SerpinA1 intron (206 bp) is a truncated α1 antitrypsin intron, whose nucleotide sequence is set forth in SEQ ID NO. 26. Modified human β-globin intron 2 is a partial sequence of modified human β-globin intron 2, whose nucleotide sequence is set forth in SEQ ID NO. 27. Modified SV40 intron is a partial sequence of simian vacuolating virus 40 intron, whose nucleotide sequence is set forth in SEQ ID NO. 28. SBR intron 3 is a modified intron of minute virus of mice, whose nucleotide sequence is set forth in SEQ ID NO. 31. MVM intron is a partial sequence of minute virus of mice intron, whose nucleotide sequence is set forth in SEQ ID NO. 34.

The above combined promoters were used to construct expression cassettes respectively, the structure of which are shown in Fig. 5. The expression cassettes were constructed into shuttle plasmids to obtain plasmid vectors ATT-PAH-WT (SEQ ID NO. 94), 100-AT-PAH-WT (SEQ ID NO. 67), ATG-PAH-WT (SEQ ID NO. 68), ATS-PAH-WT (SEQ ID NO. 69), CTS-PAH-WT (SEQ ID NO. 70) and TTM-PAH-WT (SEQ ID NO. 71).

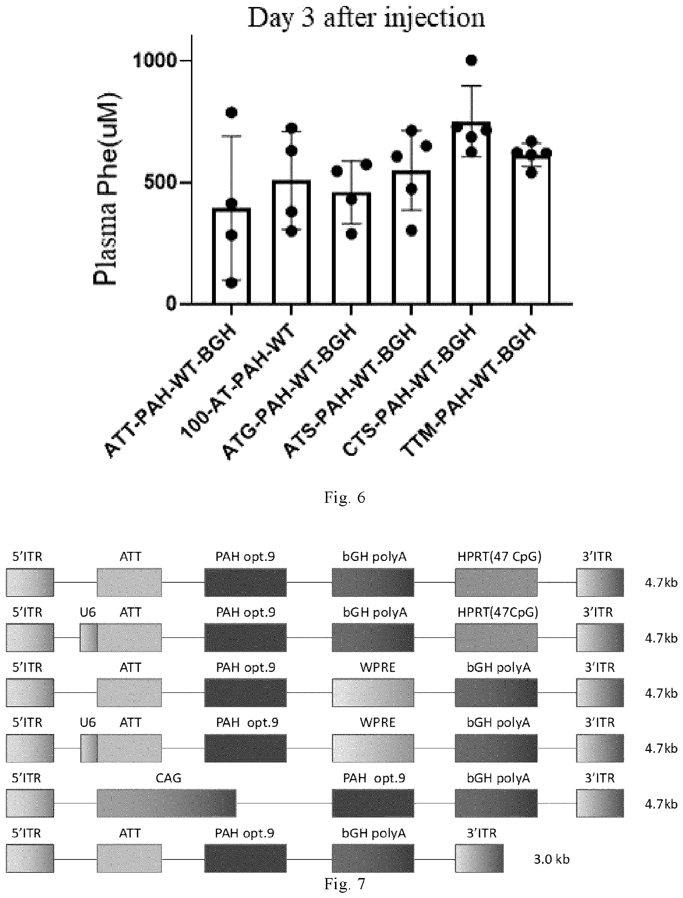

40µg of the plasmid vectors of this example were injected into PKU model mice by hydrodynamic tail vein injection. Three days after the injection, the blood of the mice was collected to determine the Phe level in the blood. The effects of different promoters in driving PAH to reduce Phe level were compared and analyzed. The results are shown in Fig. 6. The results show that the expression plasmid driven by the ATT promoter exhibits the most significant effect in reducing Phe level in PKU model mice.

**Table 1. Constituent elements of different promoters**

| Names of Combined promoter | Upstream regulatory elements, such as Enhancers | Core promoters | Introns |
|---|---|---|---|
| ATT (SEQ ID NO. 44) | ApoE HCR (321bp) (SEQ ID NO. 2) | SerpinA1 promoter (398bp) (SEQ ID NO. 3) | Truncated SerpinA1 intron (261bp) (SEQ ID NO. 4) |
| 100-AT (SEQ ID NO. 45) | ApoE HCR (321bp) (SEQ ID NO. 2) | Core SerpinA1 promoter (218 bp) (SEQ ID NOs. 24-25) | Truncated SerpinA1 intron (206bp) (SEQ ID NO. 26) |
| ATG (SEQ ID NO. 46) | ApoE HCR (321bp) (SEQ ID NO. 2) | Core SerpinA1 promoter (218 bp) (SEQ ID NOs. 24-25) | Modified human β -globin 2nd intron (184bp) (SEQ ID NO. 27) |
| ATS (SEQ ID NO. 47) | Core ApoE HCR (192bp) (SEQ ID NO. 37) | Core SerpinA1 promoter (254 bp) (SEQ ID NO.38) | Modified SV40 intron (93bp) (SEQ ID NO. 28) |
| CTS (SEQ ID NO. 48) | CRMSBS2 (72bp) (SEQ ID NO. 29) | TTRm promoter (223bp) (SEQ ID NO. 30) | SBR intron 3 (93bp) (SEQ ID NO. 31) |
| TTM (SEQ ID NO. 49) | TTR (101bp) (SEQ ID NO. 32) | TTRm promoter (228bp) (SEQ ID NO. 33) | MVM intron (77bp) (SEQ ID NO. 34) |

### Example 5: Effects of other expression regulatory elements on in vivo drug efficacy in PKU mouse model

In this example, the effects of different expression regulatory elements (U6 promoter (SEQ ID NO. 35), CAG promoter (SEQ ID NO. 50), stuffer sequence HPRT (47CpG) (SEQ ID NO. 39) and stuffer sequence WPRE (SEQ ID NO. 36)) on the Phe-reducing effect of AAV8-ATT-PAH virus were evaluated in PKU model mice. The constructions of different expression cassettes are shown in Fig. 7.

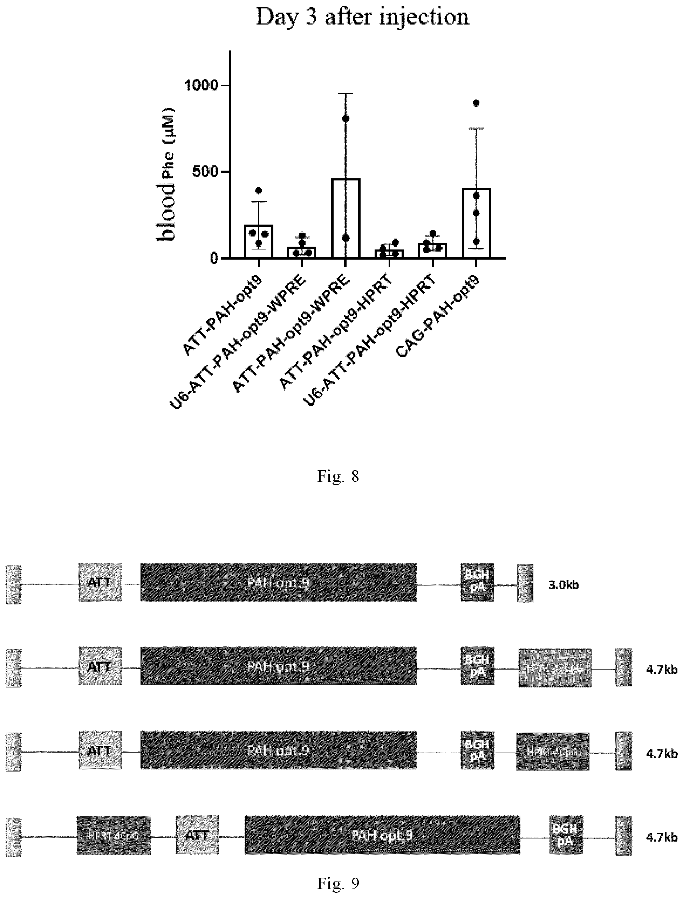

The expression cassettes shown in Fig. 7 were constructed into shuttle plasmids to obtain plasmid vectors ATT-PAH-opt9-HPRT(47CpG) (SEQ ID NO. 73), U6-ATT-PAH-opt9-HPRT(47CpG) (SEQ ID NO. 75), ATT-PAH-opt9-WPRE (SEQ ID NO. 74), U6-ATT-PAH-opt9-WPRE (SEQ ID NO. 72), CAG-PAH-opt9 (SEQ ID NO. 77), and ATT-PAH-opt9 (SEQ ID NO. 76). 40µg of the above plasmids were injected into PKU model mice by hydrodynamic tail vein injection. On the third day after injection, the blood of mice was collected to measure the Phe level in the blood. The effects of different constructed PAH expression cassettes on reducing Phe were compared and analyzed. As shown in Fig. 8, the results showed that the vectors with the stuffer sequence HPRT added (ATT-PAH-opt9-HPRT(47CpG)) exhibit better effects on reducing Phe levers than those without stuffer sequence (ATT-PAH-opt9).

### Example 6: Effect of stuff sequence optimization on in vivo efficacy in PKU mouse model

Some studies have shown that innate immune stimulation may be driven by CpG enrichment, resulting in the failure of AAV vector-mediated gene expression in vivo (Faust, Susan M et al. CpG-depleted adeno-associated virus vectors evade immune detection. The Journal of clinical investigation vol. 123,7 (2013): 2994-3001.; Konkle, Barbara A et al. BAX 335 hemophilia B gene therapy clinical trial results: potential impact of CpG sequences on gene expression. Blood vol. 137,6 (2021): 763-774.). In order to study the effect of CpG number in AAV8-ATT-PAH virus on reducing Phe level, experiments was conducted in PKU model mice, where a stuffer sequence with more CpGs (HPRT (47CpG)) and a stuffer sequence with less CpGs (HPRT (4CpG)) were used. The schematic structure of the PAH expression cassettes of the optimized recombinant AAV (rAAV) vectors, which comprised different stuffer sequences, are shown in Fig. 9. Wherein, the nucleotide sequence of HPRT (47CpG) is set forth in SEQ ID NO. 39, and that of HPRT (4CpG) is set forth in SEQ ID NO. 43.

The expression cassettes comprising different stuffer sequences were constructed into shuttle plasmids respectively. Recombinant viral vectors AAV8-ATT-PAH-opt9 (wherein the sequence of shuttle plasmid is set forth in SEQ ID NO. 76), AAV8-ATT-PAH-opt9-HPRT (47CpG) (wherein the sequence of shuttle plasmid is set forth in SEQ ID NO. 73), AAV8-ATT-PAH-opt9-HPRT (4CpG) (wherein the sequence of shuttle plasmid is set forth in SEQ ID NO. 60), and AAV8-HPRT (4CpG)-ATT-PAH-opt9 (wherein the sequence of shuttle plasmid is set forth in SEQ ID NO. 78), were obtained by using three-plasmid system of Example 1.

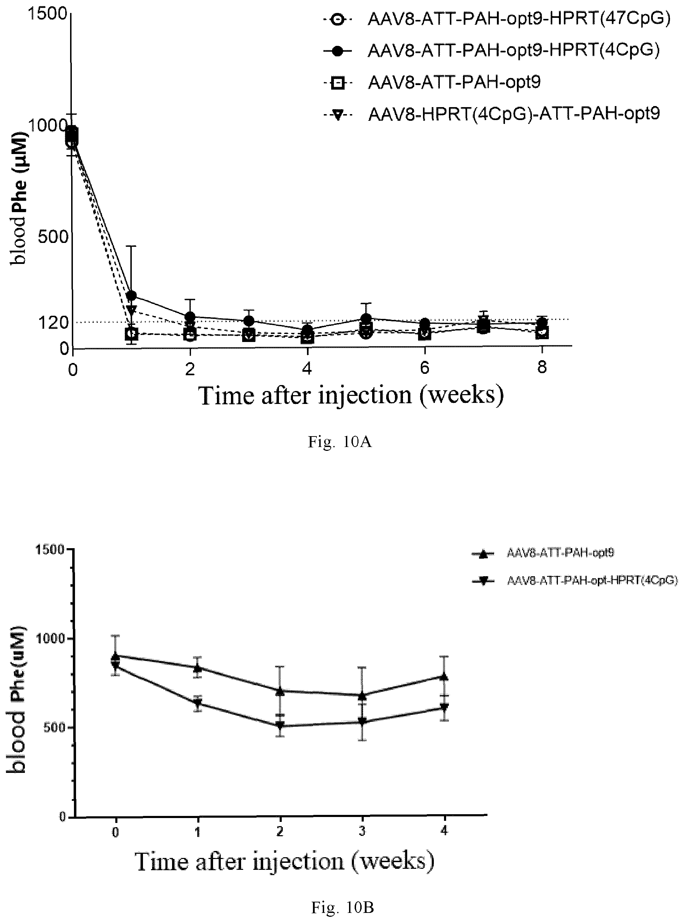

The viral vector was injected intravenously into PKU model mice at a dose of 2E11 vg/mouse. The blood of mice was collected every week after injection to measure the Phe level in the blood for 8 weeks. The effects of optimization of different stuffer sequences on reducing Phe level were compared and analyzed. As shown in Fig. 10A, after the viruses with the above four expression cassettes were injected into PKU model mice, the optimal therapeutic effects on blood Phe level of the mice were achieved (<120 µM).

In order to avoid immune stimulation caused by CpG enrichment, the expression cassette comprising the stuffer sequence with less CpGs was selected to further study the therapeutic effect of AAV8-ATT-PAH-opt9-HPRT (4CpG) on PKU model mice at a lower dose of 1E10 vg/mouse. The blood of the mice was collected every week after injection, and Phe level in the blood was measured for up to 4 weeks. The results are shown in Fig. 10B. As shown in Fig. 10B, at the low dose, the effect of AAV8-ATT-PAH-opt9 -HPRT (4CpG) on reducing Phe level is better than that of AAV8-ATT-PAH-opt9.

### Example 7: In vitro test of the optimized expression cassette AAV8-ATT-PAH-opt9-HPRT (4CpG) virus expression product PAH for its function of reducing Phe level

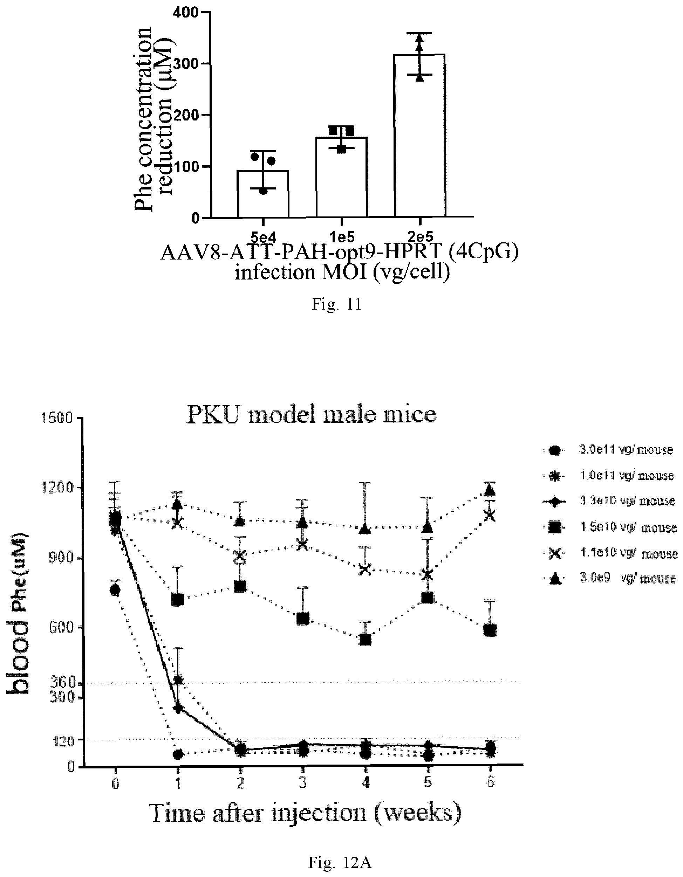

In this example, the codon-optimized AAV8-ATT-PAH-opt9-HPRT (4CpG) viral expression product was evaluated for its function of reducing Phe level in the HepG2 cell line. First, HepG2 was transiently transfected with the plasmid expressing the adeno-associated virus receptor AAVR (the AAVR gene was synthesized by GeneWei according to SEQ ID NO. 117 and then inserted in between the NheI and XbaI restriction sites into the pcDNA3.1(+) plasmid). Overexpression of AAVR will increase the efficiency of AAV8 infecting HepG2. After 24 hours, AAV8-ATT-PAH-opt9-HPRT (4CpG) virus of Example 1 was used to infect the cells at MOIs of 0, 5E4, 1E5, and 2E5, respectively. After 48 hours of culture, the Phe level was detected. The HepG2 cell line not infected with the virus was used as a reference, and the activity of PAH in reducing Phe level was evaluated by calculating the change of Phe concentration. The results shown in Fig. 11 indicated that the AAV8-ATT-PAH-opt9-HPRT (4CpG) virus expression product is capable of reducing Phe level, with a dose-dependent effect relative to the MOI of viral infection.

### Example 8: In vivo efficacy of the optimized expression cassette in the PKU mouse model: high activity at low dose

In this example, the effects of different doses of AAV8-ATT-PAH-opt9-HPRT (4CpG) virus on reducing Phe level were evaluated in male and female PKU model mice.

The AAV8-ATT-PAH-opt9-HPRT (4CpG) virus of Example 1 was injected intravenously into male PKU model mice at doses of 3.0E11, 1.0E11, 3.3E10, 1.5E10, 1.1E10, and 3.0E9 vg/mouse, respectively. Blood was collected from mice every week after injection to measure Phe level in the blood for 6 weeks. The effects of different virus administration doses on reducing Phe level were compared and analyzed. The results are shown in Fig. 12A. As shown in Fig. 12A, within 2 to 6 weeks after virus injection, Phe level in mouse blood was lower than 120µM at the dose of 3.0E11, 1.0E11, or 3.3E10 vg/mouse, indicating a therapeutic effect was achieved. Therefore, it is believed that the minimum effective dose of AAV8-ATT-PAH-opt9-HPRT (4CpG) virus for treating PKU in male mice is approximately 3.3E10 vg/mouse.

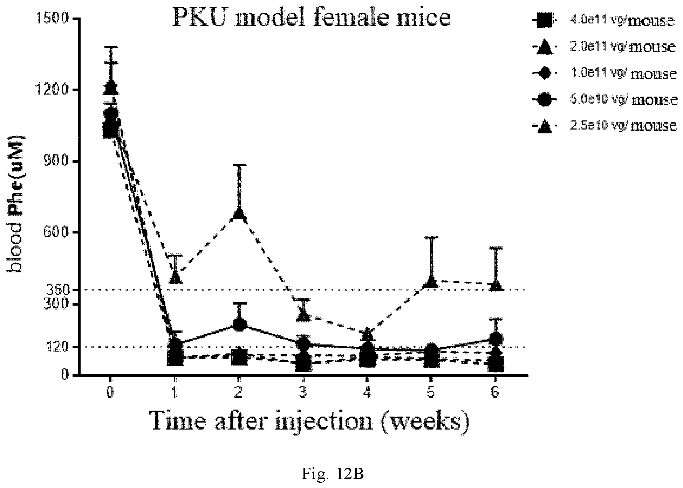

The AAV8-ATT-PAH-opt9-HPRT (4CpG) virus of Example 1 was injected intravenously into female PKU model mice at doses of 4.0E11, 2.0E11, 1.0E11, 5.0E10, and 2.5E10 vg/mouse, respectively. Blood was collected from mice every week after injection to measure the Phe level in the blood for 6 weeks. The effects of different virus administration doses on reducing Phe level were compared and analyzed. The results are shown in Fig. 12B. As shown in Fig. 12B, within 2 to 6 weeks after virus injection, Phe level in mouse blood was lower than 120µM at the dose of 4.0E11, 2.0E11, 1.0E11, and 5.0E10 vg/mouse, indicating a therapeutic effect was achieved. Therefore, it is believed that the minimum effective dose of AAV8-ATT-PAH-opt9-HPRT (4CpG) virus for treating PKU in female mice is approximately 5.0E10 vg/mouse.

### Example 9: Other efficacy of the optimized expression cassette in the PKU mouse model

In this example, other therapeutic effects of AAV8-ATT-PAH-opt9-HPRT (4CpG) virus at the dose of 3.0E10 and 3.0E11 vg/mouse on reducing PKU model mice were evaluated in male PKU model mice. Six weeks after the administration of the virus, the Tyr level in the blood was detected. The results shown in Fig. 13A demonstrates that compared with PKU heterozygous mice (Jackson Lab) without disease symptoms, the Tyr level in the blood of PKU homozygous mice with disease symptoms (i.e., PKU model mice) was significantly reduced. After receiving AAV8-ATT-PAH-opt9-HPRT (4CpG) virus at the dose of 3.0E10 and 3.0E11 vg/mouse, the Tyr level in the blood of PKU mice recovered to a level comparable to that of normal heterozygous mice. Fig. 13B shows the level of 5-hydroxyindoleacetic acid (5-HIAA, a pharmacodynamic marker for PKU patients) in the brain tissue of PKU homozygous mice, while heterozygous mice not given the drug were used as normal mouse controls. The results showed that compared with PKU heterozygous mice without disease symptoms, the 5-HIAA level in the brain tissue of PKU homozygous mice with disease symptoms was significantly reduced. After receiving AAV8-ATT-PAH-opt9-HPRT (4CpG) virus at the dose of 3.0E10 and 3.0E11 vg/mouse, the 5-HIAA level in the brain tissue of PKU mice recovered to the level comparable to that of normal heterozygous mice. In addition, the fur color of the treated PKU mice became darker and brighter. Fig. 13 C shows comparison for the fur color between the PKU homozygous mice and the vehicle group after 3 weeks of drug administration at the dose of 3.0E10 vg/mouse.

The present disclosure achieves the effect of effective, long-lasting and stable suppression of peripheral blood phenylalanine concentration in PKU mice with low dose and improves other PKU symptoms, through optimizing and screening for genes and expression regulatory elements. In addition, the present disclosure effectively reduce the dosage and possible side effects of gene therapy drugs used to treat PKU, improving the therapeutic effect.

The above description is only preferred embodiments of the present disclosure and is not intended to limit the present disclosure. Any modifications, equivalent substitutions, improvements, etc. made within the spirit and principles of the present disclosure shall be included in the scope of protection of the present disclosure.

## Claims

1. A polynucleotide molecule encoding a PAH protein, comprising a nucleotide sequence having 90% or more identity with the nucleotide sequence set forth in SEQ ID NO. 10, SEQ ID NO. 11, SEQ ID NO. 12, SEQ ID NO. 13, SEQ ID NO. 14, SEQ ID NO. 15, SEQ ID NO. 16, SEQ ID NO. 17, SEQ ID NO. 19, SEQ ID NO. 20, SEQ ID NO. 21, SEQ ID NO. 22 or SEQ ID NO. 23; preferably, a nucleotide sequence having 91%, 92%, 93%, 94%, 95%, 96%, 97%, 98%, 99% or more identity; more preferably, a nucleotide sequence having 98%, 99% or more identity;
more preferably, the polynucleotide molecule has a nucleotide sequence set forth in SEQ ID NO. 10, SEQ ID NO. 11, SEQ ID NO. 12, SEQ ID NO. 13, SEQ ID NO. 14, SEQ ID NO. 15, SEQ ID NO. 16, SEQ ID NO. 17, SEQ ID NO. 19, SEQ ID NO. 20, SEQ ID NO. 21, SEQ ID NO. 22 or SEQ ID NO. 23.

2. An expression cassette comprising the polynucleotide molecule of claim 1, and a promoter operably linked to the polynucleotide molecule;
preferably, the promoter is a specific or non-specific promoter;
preferably, the promoter is a constitutive promoter or an inducible promoter; preferably, the constitutive promoter is selected from a CMV promoter, an EF1A promoter, an EFS promoter, a CAG promoter, a CBh promoter, an SFFV promoter, an MSCV promoter, an SV40 promoter, an mPGK promoter, an hPGK promoter and a UBC promoter; preferably, the inducible promoter comprises a tetracycline-regulated promoter, an alcohol-regulated promoter, a steroid-regulated promoter, a metal-regulated promoter, a pathogenicity-regulated promoter, a temperature/heat-inducible promoter and a light-regulated promoter, and an IPTG-inducible promoter;
preferably, the promoter comprises a core promoter;
preferably, the core promoter comprises a liver-specific promoter or an active fragment thereof; preferably, the liver-specific promoter is selected from an ApoA-I promoter, an ApoA-II promoter, an ApoA-IV promoter, an ApoB promoter, an ApoC-1 promoter, an ApoC-II promoter, an ApoC-III promoter, an ApoE promoter, an albumin promoter, an alpha-fetoprotein promoter, a phosphoenolpyruvate carboxykinase (PCK1) promoter, a phosphoenolpyruvate carboxykinase 2 (PCK2) promoter, a transthyretin (TTR) promoter, an α-antitrypsin (AAT or SerpinA1) promoter, a TK (thymidine kinase) promoter, a hemoglobin promoter, an alcohol dehydrogenase 6 promoter, a cholesterol 7α-25 hydroxylase promoter, a factor IX promoter, an α-microglobulin promoter, an SV40 promoter, a CMV promoter, a Rous sarcoma virus-LTR promoter, an HBV promoter, an ALB promoter and a TBG promoter; more preferably, the liver-specific promoter is a human α1 antitrypsin promoter, preferably, the core promoter comprises a nucleotide sequence having 90% or more identity with the nucleotide sequence set forth in SEQ ID NO. 3, 24, 25, 30, 33 or 38, preferably a nucleotide sequence having 91%, 92%, 93%, 94%, 95%, 96%, 97%, 98%, 99% or more identity, and more preferably a nucleotide sequence having 98%, 99% or more identity; preferably, the core promoter has a nucleotide sequence set forth in SEQ ID NO. 3, 24, 25, 30, 33 or 38; more preferably, the nucleotide sequence of the core promoter is set forth in SEQ ID NO. 3.

3. The expression cassette according to claim 2, further comprising an expression control element, wherein the expression control element is operably linked to the polynucleotide molecule;
preferably, the expression control element is selected from at least one of a transcription/translation control signal, an enhancer, an intron, a polyA signal, an ITR, an insulator, an RNA processing signal, and an element that enhances the stability of mRNA and protein;
preferably, the expression cassette comprises a 5'ITR; preferably,the 5'ITR comprises a nucleotide sequence having 90% or more identity with the nucleotide sequence set forth in SEQ ID NO. 1, preferably a nucleotide sequence having 91%, 92%, 93%, 94%, 95%, 96%, 97%, 98%, 99% or more identity, more preferably a nucleotide sequence having 98%, 99% or more identity; more preferably, the 5'ITR has the nucleotide sequence set forth in SEQ ID NO. 1;
preferably, the expression cassette comprises a 3'ITR; preferably, the 3'ITR comprises a nucleotide sequence having 90% or more identity with the nucleotide sequence set forth in SEQ ID NO. 8, preferably a nucleotide sequence having 91%, 92%, 93%, 94%, 95%, 96%, 97%, 98%, 99% or more identity, more preferably a nucleotide sequence having 98%, 99% or more identity; more preferably, the 3'ITR has the nucleotide sequence set forth in SEQ ID NO. 8;
preferably, the expression cassette further comprises an enhancer; preferably, the enhancer is selected from an ApoE HCR enhancer or an active fragment thereof, a CRMSBS2 enhancer or an active fragment thereof, a TTRm enhancer or an active fragment thereof, and an CMV enhancer or an active fragment thereof; more preferably, the enhancer comprises a nucleotide sequence having 90% or more identity with the nucleotide sequence set forth in SEQ ID NO. 2, 29, 32, 37 or 40, preferably a nucleotide sequence having 91%, 92%, 93%, 94%, 95%, 96%, 97%, 98%, 99% or more identity, and more preferably a nucleotide sequence having 98%, 99% or more identity; preferably, the enhancer has a nucleotide sequence set forth in SEQ ID NO. 2, 29, 32, 37 or 40; more preferably, the nucleotide sequence of the enhancer is set forth in SEQ ID NO. 2;
preferably, the expression cassette further comprises an intron; preferably, the intron is selected from a truncated α1 antitrypsin intron or an active fragment thereof, a β-globin 2 intron or an active fragment thereof, an SV40 intron or an active fragment thereof, and an intron of a minute virus of mice or an active fragment thereof; preferably, the intron comprises a nucleotide sequence having 90% or more identity with the nucleotide sequence set forth in SEQ ID NO. 4, 26, 27, 28, 31 or 34, preferably a nucleotide sequence having 91%, 92%, 93%, 94%, 95%, 96%, 97%, 98%, 99% or more identity, and more preferably a nucleotide sequence having 98%, 99% or more identity; preferably, the intron has a nucleotide sequence as shown in SEQ ID NO. 4, 26, 27, 28, 31 or 34; more preferably, the nucleotide sequence of the intron is set forth in SEQ ID NO. 4;
preferably, the promoter of the expression cassette is a combined promoter comprising an upstream regulatory element, a core promoter and an intron; preferably, the upstream regulatory element is an enhancer or an active fragment thereof;
preferably, the combined promoter comprises a nucleotide sequence having 90% or more identity with the nucleotide sequence set forth in SEQ ID NO. 44, 45, 46, 47, 48 or 49, preferably a nucleotide sequence having 91%, 92%, 93%, 94%, 95%, 96%, 97%, 98%, 99% or more identity, and more preferably a nucleotide sequence having 98%, 99% or more identity; preferably, the combined promoter has a nucleotide sequence set forth in SEQ ID NO. 44, 45, 46, 47, 48 or 49; more preferably, the nucleotide sequence of the combined promoter is set forth in SEQ ID NO. 44;
preferably, the expression cassette further comprises a polyA signal; preferably, the polyA signal is at least one of bovine growth hormone poly A (BGH poly A), short poly A, SV40 polyA, and human β-globin poly A; preferably, the polyA signal comprises a nucleotide sequence having 90% or more identity with the nucleotide sequence set forth in SEQ ID NO. 7, preferably a nucleotide sequence having 91%, 92%, 93%, 94%, 95%, 96%, 97%, 98%, 99% or more identity, more preferably a nucleotide sequence having 98%, 99% or more identity; more preferably, the nucleotide sequence of the polyA signal is set forth in SEQ ID NO. 7;
preferably, the expression cassette comprises an optimized stuffer sequence; preferably, the stuffer sequence is selected from a partial intron sequence of hypoxanthine phosphoribosyltransferase (HPRT) and a Woodchuck Hepatitis Virus Posttranscriptional Regulatory Element (WPRE); preferably, the number of CpG sequences contained in the partial intron sequence does not exceed 100, 80, 60, 50, 40, 30, 20, 15, 10, 9, 8, 7, 6, 5, 4, 3, 2 or 1;
preferably, the partial intron sequence or a Woodchuck Hepatitis Virus Posttranscriptional Regulatory Element (WPRE) does not contain a CpG sequence; preferably, the stuffer sequence is a partial intron sequence of hypoxanthine phosphoribosyltransferase (HPRT); preferably, the stuffer sequence comprises a nucleotide sequence having 90% or more identity with the nucleotide sequence set forth in SEQ ID NO. 39 or SEQ ID NO. 43, preferably a nucleotide sequence having 91%, 92%, 93%, 94%, 95%, 96%, 97%, 98%, 99% or more identity, and more preferably a nucleotide sequence having 98%, 99% or more identity; more preferably, the nucleotide sequence of the filler sequence is set forth in SEQ ID NO. 39 or SEQ ID NO. 43; and
preferably, the expression cassette comprises a Kozak start sequence; the Kozak start sequence comprises a nucleotide sequence having 90% or more identity with the nucleotide sequence set forth in SEQ ID NO. 5, preferably a nucleotide sequence having 91%, 92%, 93%, 94%, 95%, 96%, 97%, 98%, 99% or more identity, and more preferably a nucleotide sequence having 98%, 99% or more identity; more preferably, the Kozak start sequence has the nucleotide sequence set forth in SEQ ID NO. 5.

4. The expression cassette according to claim 2 or 3, comprising a 5'ITR, an ApoE HCR enhancer, a human α1 antitrypsin promoter, a truncated α1 antitrypsin intron, a Kozak start sequence, the polynucleotide molecule, a BGH poly A, a partial intron sequence of hypoxanthine phosphoribosyltransferase (HPRT) and a 3'ITR; preferably, the expression cassette comprises a nucleotide sequence having 90% or more identity with the nucleotide sequence sset forth in SEQ ID NO. 80, SEQ ID NO. 81, SEQ ID NO. 82, SEQ ID NO. 83, SEQ ID NO. 84, SEQ ID NO. 85, SEQ ID NO. 86, SEQ ID NO. 87, SEQ ID NO. 89, SEQ ID NO. 90, SEQ ID NO. 91, SEQ ID NO. 92 or SEQ ID NO. 93, preferably a nucleotide sequence having 91%, 92%, 93%, 94%, 95%, 96%, 97%, 98%, 99% or more identity, and more preferably a nucleotide sequence having 98%, 99% or more identity; more preferably, the expression cassette has a nucleotide sequence set forth in SEQ ID NO.80, SEQ ID NO. 81, SEQ ID NO. 82, SEQ ID NO. 83, SEQ ID NO. 84, SEQ ID NO. 85, SEQ ID NO. 86, SEQ ID NO. 87, SEQ ID NO. 89, SEQ ID NO. 90, SEQ ID NO. 91, SEQ ID NO. 92 or SEQ ID NO. 93.

5. An expression vector comprising the polynucleotide molecule of claim 1 or the expression cassette of any one of claims 2 to 4; preferably, the expression vector further comprises a gene encoding a marker, preferably, the marker is selected from at least one of an antibiotic resistance protein, a toxin resistance protein, a colored or fluorescent or luminescent protein, and a protein that mediates enhanced cell growth and/or gene amplification;
preferably, the antibiotic is selected from at least one of ampicillin, neomycin, G418, puromycin and blasticidin;
preferably, the toxin is selected from at least one of anthrax toxin and diphtheria toxin; preferably, the colored or fluorescent or luminescent protein is selected from at least one of green fluorescent protein, enhanced green fluorescent protein, red fluorescent protein and luciferase;
preferably, the protein that mediates enhanced cell growth and/or gene amplification is dihydrofolate reductase (DHFR); and
preferably, the expression vector comprises an origin of replication; preferably, the origin of replication sequence is selected from at least one of f1 phage ori, RK2oriV, pUC ori and pSC101ori.

6. The expression vector according to claim 5, which is selected from a plasmid, a cosmid, a viral vector, an RNA vector or a linear or circular DNA or RNA molecule;
preferably, the plasmid is selected from pCI, puc57, pcDNA3, pSG5, pJ603 or pCMV; preferably, the viral vector is selected from a retrovirus, an adenovirus, a parvovirus (e.g., an adeno-associated virus), a coronavirus, a negative-strand RNA virus such as an orthomyxovirus (e.g., influenza virus), a rhabdovirus (e.g., rabies and vesicular stomatitis virus), a paramyxovirus (e.g., mammary gland and Sendai), a positive-strand RNA virus (such as a picornavirus and an alphavirus), or a double-stranded DNA virus, the double-stranded DNA virus is selected from an adenovirus, a herpesvirus (e.g., herpes simplex virus type 1 and 2, Epstein-Barr virus, cytomegalovirus), a poxvirus (e.g., vaccinia virus, fowlpox virus, and canarypox virus), a Norwalk virus, a togavirus, a flavivirus, a reovirus, a papovavirus, a hepadnavirus, a baculovirus, or a hepatitis virus;
preferably, the retrovirus is selected from avian leukocytosis-sarcoma, mammalian C-type, B-type virus, D-type virus, HTLV-BLV collection, lentivirus or foamy virus; and
preferably, the lentiviral vector is selected from HIV-1, HIV-2, SIV, FIV, BIV, EIAV, CAEV or ovine demyelinating leukoencephalitis lentivirus.

7. The expression vector according to claim 5 or 6, which is an adeno-associated virus vector;
preferably, the adeno-associated virus is selected from AAV type 1, AAV type 2, AAV type 3, AAV type 4, AAV type 5, AAV type 6, AAV type 7, AAV type 8, AAV type 9, AAV type 10, avian AAV, bovine AAV, canine AAV, equine AAV, or ovine AAV; and
preferably, the expression vector comprises a nucleotide sequence having 90% or more identity with the nucleotide sequence set forth in SEQ ID NO. 53, SEQ ID NO. 54, SEQ ID NO. 55, SEQ ID NO. 56, SEQ ID NO. 57, SEQ ID NO. 58, SEQ ID NO. 59, SEQ ID NO. 60, SEQ ID NO. 62, SEQ ID NO. 63, SEQ ID NO. 64, SEQ ID NO. 65, SEQ ID NO. 66, SEQ ID NO. 72, SEQ ID NO. 73, SEQ ID NO. 74, SEQ ID NO. 75, SEQ ID NO. 76, SEQ ID NO. 77, SEQ ID NO. 78, SEQ ID NO. 96, SEQ ID NO. 97, SEQ ID NO. 98, SEQ ID NO. 99, SEQ ID NO. 100, SEQ ID NO. 101, SEQ ID NO. 102, SEQ ID NO. 103, SEQ ID NO. 105, SEQ ID NO. 106, SEQ ID NO. 107, SEQ ID NO. 108 or SEQ ID NO. 109, preferably a nucleotide sequence having 91%, 92%, 93%, 94%, 95%, 96%, 97%, 98%, 99% or more identity, and more preferably a nucleotide sequence having 98%, 99% or more identity; more preferably, the expression vector has a nucleotide sequence set forth in SEQ ID NO. 53, SEQ ID NO. 54, SEQ ID NO. 55, SEQ ID NO. 56, SEQ ID NO. 57, SEQ ID NO. 58, SEQ ID NO. 59, SEQ ID NO. 60, SEQ ID NO. 62, SEQ ID NO. 63, SEQ ID NO. 64, SEQ ID NO. 65, SEQ ID NO. 66, SEQ ID NO. 72, SEQ ID NO. 73, SEQ ID NO. 74, SEQ ID NO. 75, SEQ ID NO. 76, SEQ ID NO. 77, SEQ ID NO. 78, SEQ ID NO. 96, SEQ ID NO. 97, SEQ ID NO. 98, SEQ ID NO. 99, SEQ ID NO. 100, SEQ ID NO. 101, SEQ ID NO. 102, SEQ ID NO. 103, SEQ ID NO. 105, SEQ ID NO. 106, SEQ ID NO. 107, SEQ ID NO. 108 or SEQ ID NO. 109; more preferably, the expression vector has a nucleotide sequence set forth in SEQ ID NO. 76, SEQ ID NO. 73, SEQ ID NO. 60 or SEQ ID NO. 78.

8. A viral particle comprising at least one of the polynucleotide molecule of claim 1, the expression cassette of any one of claims 2 to 4, and the expression vector of any one of claims 5 to 7.

9. A pharmaceutical composition for treating phenylketonuria, comprising at least one of the polynucleotide molecule of claim 1, the expression cassette of any one of claims 2 to 4, the expression vector of any one of claims 5 to 7 and the viral particle of claim 8; optionally, the pharmaceutical composition further comprises a pharmaceutically acceptable carrier; wherein the pharmaceutical composition expresses wild-type or codon-optimized PAH protein.

10. The use of at least one of the polynucleotide molecule of claim 1, the expression cassette of any one of claims 2 to 4, the expression vector of any one of claims 5 to 7, the virus particle of claim 8 and the pharmaceutical composition of claim 9 in the preparation of a medicament for treating phenylketonuria.
